(19) Europäisches Patentamt
European Patent Office
Office européen des brevets

(11) **EP 3 339 288 A1**

(12) **EUROPÄISCHE PATENTANMELDUNG**

(43) Veröffentlichungstag:
27.06.2018 Patentblatt 2018/26

(51) Int Cl.:
*C07C 291/04* (2006.01)  *C07D 295/24* (2006.01)

(21) Anmeldenummer: **16206623.7**

(22) Anmeldetag: **23.12.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Aurotec GmbH**
**4844 Regau (AT)**

(72) Erfinder: **Die Erfindernennung liegt noch nicht vor**

(74) Vertreter: **Sonn & Partner Patentanwälte**
**Riemergasse 14**
**1010 Wien (AT)**

(54) **HERSTELLUNG EINES AMINOXIDS DURCH OXIDATION EINES TERTIÄREN AMINS**

(57) Die Erfindung betrifft ein Verfahren zur Herstellung eines Aminoxids durch Oxidation eines tertiären Amins in einem Reaktor unter kontinuierlicher Einführung von tertiärem Amin in einem Reaktionsfluid und Ausführung von Aminoxid, wobei ein geeignetes Oberfläche-zu-Volumenverhältnis bzw. eine passende Strömungsgeschwindigkeit mit entsprechenden Oberflächen/Volumenbelastungen im kontinuierlichen Prozess gewählt werden.

EP 3 339 288 A1

**Beschreibung**

Die vorliegende Erfindung betrifft das Gebiet der Oxidation von tertiären Aminen zu Aminoxiden.

**Hintergrund der Erfindung**

Zellulose kann in wässrigen Lösungen von Aminoxiden, z.B. von Lösungen von N-Methyl-Morpholin-N-oxid (NMMO oder NMMNO), gelöst werden, um aus der erhaltenen Spinnlösung Spinnprodukte, wie zum Beispiel Filamente, Stapelfaser, Folien, etc. herzustellen (WO 2013/030400, US 2,179,181, US 3,447,939). Daher besteht ein wachsender Bedarf an Aminoxiden wie NMMO. Weitere Anwendungen von tertiären Aminoxiden sind oberflächenaktive Mittel, Bakteriostatika, Shampoos und Waschmittel.

Die DE 2557456 A1 beschreibt ein Verfahren zur Herstellung von Aminoxiden, wobei ein tertiäres Amin mit Wasserstoffperoxid in Gegenwart von Wasser umgesetzt wird. Bei einer Aminoxidkonzentration von über 30% steigt die Viskosität des Produktes stark an, wodurch die weitere Verarbeitung erschwert wird. Es wurde erkannt, dass bei Aminoxidkonzentration über 60% ein Viskositätsminimum erreicht wird.

Die US 3,447,939 beschreibt die diskontinuierlich betriebene Batch Herstellung von N-Methyl-Morpholin-N-Oxide (NMMNO), wobei das Edukt N-Methylmorpholin (NMM) in Gegenwart von Wasser und 35%igem Wasserstoffperoxid, bei den gewählten Reaktionstemperaturen von 67-72°C über einen Zeitraum von 4-5 Stunden in das entsprechende tertiäre Aminoxid NMMNO umgesetzt wird. Die Zersetzung des überschüssigen Wasserstoffperoxides erfolgt enzymatisch mit Katalase. Das gebildete Reaktionsprodukt wurde durch azeotrope Benzol-Wasser Destillation und Umkristallisation gereinigt.

In der DD 246997 A1 wird ein ähnliches Batch-Verfahren beschrieben, in dem Wasserstoffperoxid (45-50%) über einen Sprühteller oder eine Einspritzvorrichtung in feinverteilter Form unter starkem Rühren in das Edukt NMM eingebracht wird.

In dem Verfahren nach DE 3618352 A1 wird nach einer Oxidation mittels Wasserstoffperoxid über 4-6 Stunden verbleibendes Peroxid durch Katalase abgebaut.

EP 0 307 184 A2 greift mit Bezug auf das US Patent 4,247,480 das Problem der Nitrosaminbildung während der Umsetzung von tertiärem Amin zum entsprechenden Aminoxid auf und schlägt vor, dass bei Einsatz von Kohlendioxid Atmosphäre (0,03-3 MPa) die Reaktion bei niedrigen Temperaturen <45°C und Normaldruck durchgeführt werden soll.

Da die Nitrosaminbildung auch von ubiquitär vorhandenen Stickoxiden der Luft beeinflusst werden kann schlägt US 4,994,614 A vor, den unbefüllten Raum des Reaktionskessels mit Kohlendioxid zu überlagern, damit die Stickoxide der Luft ferngehalten werden. Solch eine Spül- und Überlagerungsmaßnahme ist sehr aufwändig, da die Reaktionsbehälter vor der Zugabe der Edukte, während der Reaktion, nach Anschluss der Reaktion und während der Behälterentleerung mit Kohlendioxid gespült bzw. überlagert werden müssen.

US 5,216,154 A schlägt zur Reduzierung sich bildender Nitrosamine vor, dass die Umsetzung vom tertiären N-Methylmorpholin (NMM) zu NMMNO im Rührkessel, unter einer bestimmten Eduktgemischzusammensetzung und speziellen Verfahrensparametern durchgeführt werden soll.

Es ist ein Ziel der vorliegenden Erfindung einen skalierbaren Prozess zur Herstellung von Aminoxiden zur Verfügung zu stellen, der eine hohe Ausbeute, geringe bis keine Neben- und Abbauprodukte und eine effiziente Umwandlung ermöglicht.

**Zusammenfassung der Erfindung**

Die vorliegende Erfindung betrifft ein Verfahren zur Herstellung eines Aminoxids durch Oxidation eines tertiären Amins in einem Reaktor mit einem oder mehreren länglichen Hohlkörper als Reaktionsbereich, wie z.B. ein Rohr, wobei der Reaktor vorzugsweise ein Reaktionsrohr ist, unter kontinuierlicher Einführung von tertiärem Amin in einem Reaktionsfluid und Ausführung von Aminoxid. Dazu wurden geeignete Prozessparameter, insbesondere ein geeignetes Oberfläche-zu-Volumenverhältnis bzw. eine passende Strömungsgeschwindigkeit mit entsprechenden Oberflächen/Volumenbelastungen im kontinuierlichen Prozess gewählt. Insbesondere ist im Hohlkörper i) ein Oberfläche zu Volumenverhältnis von 0,5 m$^2$/m$^3$ oder mehr - zumindest auf einer Länge des Hohlkörpers, in der das Amin zu 50% oxidiert wird, ii) eine spezifische Oberflächenbelastung von 1 l/m$^2$h bis 40 l/m$^2$h, und/oder iii) eine spezifische Volumenbelastung von 1.000 l/m$^3$h bis 30.000 l/m$^3$h, vorhanden. Diese drei Kennzeichen i), ii) und iii) betreffen im Grunde alternative Definitionen, welche das erfindungsgemäße Verfahren beschreiben können. In manchen Ausführungsformen treffen alle Parameter i), ii) und iii) zu. Die folgende ausführliche Beschreibung und insbesondere die vorzugsweisen Ausführungsformen betreffen alle drei Parameter i), ii) und iii) gleichermaßen. Alle erfindungsgemäßen Ausführungsformen und Aspekte sind miteinander kombinierbar, und wurden dies auch, wie in den experimentellen Beispielen gezeigt wurde.

**Kurzbeschreibung der Figuren**

Figur 1 zeigt den Zusammenhang von Verweilzeit im Hohlkörper und Umsatz für den Fall der Oxidation von NMM.

Figur 2 zeigt einen Mikroreaktor (1) mit Zufuhr der Edukte (3,4) über die Pumpen (5,6) und Ableitung nach dem Druckhalteventil (11) zum Produktbehälter (12).

Figur 3 zeigt den Ausbeuteverlauf bei unterschiedlicher $CO_2$-Beimischung.

Figur 4 zeigt den Umsatzverlauf bei unterschiedlicher $CO_2$-Beimischung.

Figur 5 zeigt Umsätze in einem Reaktor aus Hastelloy® C-22.

Figur 6 zeigt die Stabilisierung des Wasserstoffperoxids mit Phosphorsäure zur Reaktionsverbesserung.

Figur 7 zeigt den Einfluss der $CO_2$ Konzentration auf die Reaktionsgeschwindigkeit.

Figur 8 zeigt den Einfluss der Temperatur auf den Reaktionsfortschritt, Umsatz-Ausbeuteverlauf bei 30 Gew.% $H_2O_2$, 1 Gew.% $CO_2$, 0,1 Gew.% $H_3PO_4$.

Figur 9 zeigt den Einfluss der Konzentration des Wasserstoffperoxids auf die Produktionsrate.

Figur 10 zeigt Ergebnisse mit einem Großvolumenreaktor.

Figur 11 zeigt einen Mikroreaktor analog zu Fig. 2 mit zusätzlicher Zuleitung für ein 3. Edukt (z.B. $CO_2$).

Figur 12 zeigt Ergebnisse zur separaten $CO_2$-Zufuhr.

**Detaillierte Beschreibung der Erfindung**

Die Erfindung betrifft ein Verfahren zur Herstellung eines Aminoxids durch Oxidation eines tertiären Amins in einem Reaktor unter kontinuierlicher Einführung von tertiärem Amin in einem Reaktionsfluid und Ausführung von Aminoxid, wobei ein geeignetes Oberfläche-zu-Volumenverhältnis bzw. eine passende Strömungsgeschwindigkeit mit entsprechenden Oberflächen/Volumenbelastungen im kontinuierlichen Prozess gewählt werden. Der kontinuierliche Prozess wird in einem länglichen Hohlkörper, mit einem länglichen Bereich wischen einem Einlass und Auslass durchgeführt. Die erfindungsgemäßen Parameter und baulichen Merkmale finden sich zwischen Ein- und Auslass, also in der Reaktions- oder Reaktorzone. Der Reaktor ist insbesondere ein milli- oder mikrostrukturierter Reaktor oder Apparat. Der Hohlköper ist insbesondere bevorzugt ein Rohr. Insbesondere ist im Hohlkörper i) ein Oberfläche zu Volumenverhältnis von 0,5 m²/m³ oder mehr - zumindest auf einer Länge des Hohlkörpers, in der das Amin zu 50% oxidiert wird, ii) eine spezifische Oberflächenbelastung von 1 l/m²h bis 40 l/m²h, und/oder iii) eine spezifische Volumenbelastung von 1.000 l/m³h bis 30.000 l/m³h, vorhanden.

Bisher werden tertiäre Aminoxide für kleinere Mengen im Labor oder für größere Mengen in Chargenprozessen hergestellt. Es ist ein Ziel für Großanlagen ein möglichst kontinuierliches Verfahren zum Einsatz kommen zu lassen, da dieses eine effizientere Reaktionsführung zulässt und höhere Produktivitäten durch geringere Stillstandszeiten erreicht werden, da dadurch unnötige Reinigungsschritte vor dem nächsten Chargenprozess vermieden werden. Zudem hat sich gezeigt, dass Schutzgase ($CO_2$) in Chargenprozessen notwendig sind und der Reaktionsflüssigkeit überlagert werden müssen. Im erfindungsgemäßen milli- oder mikrostrukturierten Apparat erübrigt sich dieses Problem, da das Schutzgas direkt in die Rohre eingebracht werden kann, ohne, dass ein Gasbereich entsteht oder schädlich wäre. Erfindungsgemäß lassen sich sowohl Druck als auch Temperatur besser kontrollieren.

Zudem ist es in einem kontinuierlichen Reaktor möglich, das durchgehend bewegte Reaktionsfluid in einzelne Bereiche zu unterteilen, in denen die Reaktion in unterschiedlichen Fortschrittsstadien ist. Diese Bereiche können unterschiedlich hinsichtlich Druck und/oder Temperatur bzw. Temperierung behandelt werden. Solche Bereiche sind insbesondere der erste Bereich, in dem bis zu 50% (mol.-%) des tertiären Amins oxidiert werden (schnelle Reaktion, hohe Exothermie) und der zweite Bereich, in dem der Rest des tertiären Amins (bis zur gewünschten Ausbeute) oxidiert wird (langsamere Reaktion, geringere Exothermie).

"Kontinuierliche" Reaktion oder Prozessführung bedeutet, dass laufend während der Reaktion Ausgangsstoff, also Fluid mit dem tertiären Amin zugeführt wird, und dieses daraufhin laufend im Reaktor oxidiert wird. Laufend bedeutet nun nicht, dass der Prozess nicht gestoppt werden darf, sondern bloß, dass während der Herstellung von Aminoxid die Edukte mehr oder weniger gleichmäßig zugeführt werden. Vorzugsweise ist die kontinuierliche Zuführung während der Oxidationsreaktion ununterbrochen. Alternativ, aber weniger bevorzugt, kann auch eine absatzweise und/oder periodische Zufuhr gewählt werden. Gesagtes zur Zuführung gilt ebenso für eine Abfuhr des Produkts, also des Aminoxids.

Der Reaktor ist üblicherweise ein milli- oder mikrostrukturierter Apparat und/oder ein Reaktor mit einem länglichen Hohlkörper als Reaktionskörper, vorzugsweise ein rohrartiger Mikroreaktor. Ein geeigneter Mikroreaktor ist beispielsweise in der WO 2010/055034 (unter Bezugnahme hierin aufgenommen) offenbart. Ein Reaktor kann ein oder mehrere parallele längliche Hohlkörper enthalten, in denen die Reaktion durchgeführt wird. Der Definitionen des Begriffs "Hohlkörper" werden verwendete um die Gesamtheit der Hohlkörper für die Reaktion zu beschreiben - sofern nicht zu anderen Zwecken vorgesehen, wie dem Vorheizen. In den Hohlkörpern wird das Reaktionsfluid befördert, welches durch den Einlass des Ausgangsstoffes (bzw. der Ausgangsstoffe) gesteuert wird. Das Fluid wird als Reaktionsfluid bezeichnet, sobald alle zur Oxidation nötigen Bedingungen bestehen (alle Substanzen zur Reaktion vorhanden, Temperatur, Druck).

Der oder die Hohlkörper eines geeigneten Reaktors kann/können beliebig auf die nötigen Dimensionen skaliert werden. Allerdings muss bei der exothermen Oxidation von tertiären Aminen auf die nötige Temperierung, d.h. Wärmeabfuhr geachtet werden. Hierzu ist ein Reaktor, wobei der Hohlkörper von einem Temperierfluid umspült wird, bestens geeignet. Ein derartiger Reaktor ist in WO 2010/055034 beschrieben. Es hat sich gezeigt, dass im Fall der erfindungsgemäßen Oxidationsreaktion bestimmte Dimensionen vorteilhaft sind. Insbesondere beträgt vorzugsweise der Innendurchmesser eines Hohlkörpers des Reaktors 0,25 mm bis 10 mm, vorzugsweise 0,5 mm bis 6 mm, insbesondere bevorzugt 0,8 mm bis 4 mm. Die Länge eines Hohlkörpers des Reaktors ist vorzugsweise 0,5 m bis 20 m, bevorzugt 1 m bis 10 m, insbesondere 2 m bis 5 m. Auch andere Geometrien sind möglich. Bei dickeren Hohlkörpern kann eine größere Oberfläche zum erhöhten Temperaturaustausch notwendig sein. Der Temperaturaustausch des innengeführten Reaktionsfluids kann auch dadurch verbessert werden, dass die Edukte in niedrigerer Konzentration zugeführt werden, oder durch höhere Strömungsgeschwindigkeiten des Rektionsfluids, oder über das außen geführte Temperierfluid durch Erhöhen der Strömungsgeschwindigkeit, oder die Wahl eines Temperierfluids mit höherer Wärmekapazität.

Zur Oxidation des tertiären Amins kann ein Oxidationsmittel zugeführt werden. Eine Gruppe von Oxidationsmitteln sind beispielsweise Peroxide, wobei Wasserstoffperoxid am meisten bevorzugt ist. Andere Peroxide sind z.B. Percarbonsäuren. Das tertiäre Amin wird vorzugsweise mit einem Molverhältnis zum Oxidationsmittel von 1:0,9 bis 1:1,3, vorzugsweise von 1:1 bis 1:1,1, eingesetzt.

In besonders bevorzugten Ausführungsformen ist das Aminoxid N-Methyl-Morpholin-N-oxid, welches durch Oxidation von N-Methylmorpholin erhalten werden kann. Letzteres ist somit ein bevorzugtes tertiäres Amin.

Erfindungsgemäß hat sich gezeigt, dass die Oxidation des Reaktionsfluids in einem Reaktor wie einem milli- oder mikrostrukturierten Apparat bei einem Oberfläche zu Volumenverhältnis von 0,5 $m^2/m^3$ oder mehr - zumindest auf einer Länge des Hohlkörpers, in der das tertiäre Amin zu 50% (mol.-%) oxidiert wird (erster Bereich, wie oben erklärt), gut durchgeführt werden kann. Die Oberfläche kann durch die Innenfläche des Hohlkörpers bestimmt werden. Insbesondere bevorzugt sind höhere Oberfläche zu Volumenverhältnisse, z.B. von 1 $m^2/m^3$ oder mehr, 1,5 $m^2/m^3$ oder mehr, oder 2 $m^2/m^3$ oder mehr. Bei diesen Dimensionen können sehr effizient, die Prozessparameter wie Fließgeschwindigkeit und Temperatur bei der Oxidation von tertiären Aminen in einem kontinuierlichen Prozess gesteuert werden. Durch dieses im Vergleich zu Batch-Verfahren hohe Oberfläche zu Volumenverhältnis können große volumensbezogene Wärmeströme (Kühlung und Erwärmung der Edukte und des Reaktionsgesmisches) und intensives Mischen ermöglicht werden. Diese intensive Mischung wiederum stellt sicher, dass die Reaktion sehr spezifisch und vollständig abläuft.

Ein weiterer Parameter zur Charakterisierung des erfinderischen Verfahrens ist die spezifische Oberflächenbelastung, welche im Bereich von 1 $l/m^2h$ bis 40 $1/m^2h$ sein sollte, vorzugsweise von 5 $1/m^2h$ bis 30 $l/m^2h$ oder von 10 $l/m^2h$ bis 20 $l/m^2h$. Die spezifische Volumenbelastung weist einen alternativen oder weiteren Kennwert des erfindungsgemäßen Verfahrens auf, welcher von 1.000 $l/m^lh$ bis 30.000 $l/m^3h$ sein sollte, vorzugsweise von 1.500 $l/m^3h$ bis 25.000 $l/m^3h$, insbesondere bevorzugt von 2.000 $l/m^3h$ bis 20.000 $l/m^3h$. Diese spezifischen Belastungen, je nach Volumen im Hohlkörper oder inneren Oberfläche des Hohlkörpers, zeigen die Dimensionierung des Reaktors bzw. des darin transportierten Fluids zur Reaktoroberfläche, mit der die Reaktionsbedingungen im Reaktionsfluid kontrolliert werden können.

Nachdem die Oxidation von tertiärem Amin zu Aminoxid einer großen Wärmetönung unterliegt, im Fall von NMM zu NMMO mit einem adiabaten Temperaturanstieg bis 400 K und einer spez. Reaktionsethalpie von bis zu -1600 kJ/kg, kann dies in besonders guter Art und Weise mit dem erfindungsgemäß dargestellten Verfahren ermöglicht werden. Die Handhabung solch extrem exothermer Reaktionen, wie die Oxidierung von NMM zu NMMNO erfordert extrem hohe volumens- und durchsatzspezifische Wärmeströme. Dies bedeutet im erfindungsgemäßen Verfahren, dass punktuelle Überhitzungen vermieden werden (Hot Spots), welche sehr leicht und unkontrolliert in Bachtreaktoren auftreten können. Die intensive Energieabfuhr des erfindungsgemäßen Verfahrens ermöglicht es, dass die Geschwindigkeit der Oxidation von tertiärem Amin in Aminoxid unter entsprechender Temperatur- und Druckanpassung angehoben werden kann, ohne dass im Rohrreaktor nennenswerte, räumlich vorherrschende Temperaturunterschiede vorkommen, die extrem betrachtet das "thermische Durchgehen" der Reaktion bedeuten könnten und sich zusätzlich negativ auf die Selektivität der Reaktion auswirken würden. Mit dem erfindungsgemäß dargestellten Verfahren wurde gezeigt, dass die Herstellung von Aminoxid unter kontrollierteren Prozesstemperaturen und höheren Drücken gegenüber dem Stand der Technik erfolgen kann.

Im zweiten Bereich, also einen Bereich nachdem bereits 50% (mol.%) des tertiären Amins oxidiert wurden, können niedrigere Oberfläche zu Volumenverhältnisse gewählt werden, wobei auch zum ersten Bereich überlappende Werte möglich sind. Vorzugsweise ist im zweiten Bereich das Oberfläche zu Volumenverhältnis 5 $m^2/m^3$ oder kleiner, besonders bevorzugt 4 $m^2/m^3$ oder kleiner, 3 $m^2/m^3$ oder kleiner oder 2 $m^2/m^3$ oder kleiner.

Selbstverständlich sind auch andere Bereichseinteilungen mit unterschiedlichen Temperaturbedingungen, insbesondere eine andere Temperierung, also ein anderer Wärme(ab)transport vom reaktionsfluid, möglich. Solche Bereichseinteilungen beziehen sich auf den Fortschritt der Oxidation des tertiären Amins, also z.B. eine Bereichseinteilung in Teile des Reaktionsfluids bis zu Reaktionsfortschrittsgrenzen an denen 25%, 33% oder 50%, des tertiären Amins oxidiert wurden.

Anhand der Flussrate (oder Strömungsgeschwindigkeit) des Reaktionsfluides (mit dem Ausgangsstoff) können bei einem entsprechend dimensionierten Hohlkörpers diverse Parameter eingestellt werden, beispielsweise die Verweilzeit des tertiären Amins im Reaktor. Die Verweilzeit ist vorzugsweise von 0,4 Minuten bis 14 Minuten, bevorzugt 0,6 Minuten bis 8 Minuten. Mittels der Verweilzeit kann auch der Reaktionsfortschritt in einzelnen Bereichen gesteuert werden. Der Zusammenhang der Verweilzeit und der Oxidation verhält sich annähernd wie eine chemische Reaktion der 2. Ordnung. Der Umsatz und die Verweilzeit einer Reaktion 2. Ordnung hängt über folgende Formel zusammen:

$$X_A = \frac{\tau c_{A,0} k}{1 + \tau c_{A,0} k}$$

k......Geschwindigkeitskonstante der chemischen Reaktion in $lmol^{-1}s^{-1}$
$c_{A,0}$... Ausgangskonzentration vom Edukt A (tertiäres Amin) in $moll^{-1}$
τ......Verweilzeit in s
$X_A$......Umsatz des Eduktes A (tertiäres Amin) als Anteil

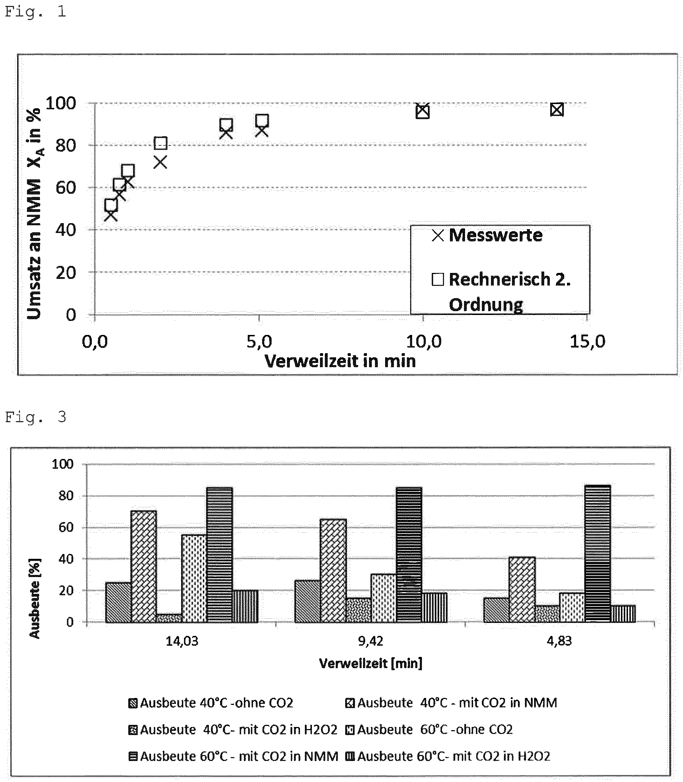

Derartige Verweilzeiten zu Umsätzen sind für den Fall von NMM in Fig. 1 dargestellt. Wie gesagt, können Verweilzeiten über die Strömungsgeschwindigkeit eingestellt werden. Die Strömungsgeschwindigkeit beeinflusst auch die Temperierung und die Einteilung des Reaktor in entsprechende Bereiche, da mit Fortschreiten der Reaktion tertiäres Amin verbraucht wird, seine Konzentration daher sinkt und folglich auch die Reaktion sich verlangsamt, womit in weiter Folge auch weniger Wärme abgeführt wird. Eine bevorzugte Strömungsgeschwindigkeit des Reaktionsfluids, die für alle Faktoren geeignet ist, ist 0,1 m/min bis 60 m/min, bevorzugt 10 m/min bis 40 m/min, am meisten bevorzugt 20 m/min bis 25 m/min.

Es ist auch möglich unterschiedliche Strömungsgeschwindigkeiten in unterschiedlichen Bereichen (insbesondere wie oben definiert, je nach Reaktionsfortschritt) auszuwählen. Unterschiedliche Strömungsgeschwindigkeiten ergeben sich bei unterschiedlichen Hohlkörperquerschnitten in den unterschiedlichen Bereichen.

Zusätzlich zur Kontrolle ist somit alternativ oder in Kombination eine unterschiedliche Reaktionsgeschwindigkeit in einzelnen Bereichen steuerbar.

Vorzugsweise ist die spezifische Reaktionswärme zwischen 540 kJ/kg und 1610 kJ/kg, bevorzugt zwischen 1110 kJ/kg und 1420 kJ/kg. Dieser Wert wird insbesonders bevorzugt im ersten Bereich (Reaktion bis 50% des tertiären Amins, wie oben definiert) eingehalten.

Höhere Reaktionsgeschwindigkeiten können bei der Aminoxidherstellung durch Erhöhung der Prozesstemperatur erreicht werden. Allerdings besteht aufgrund der starken Exothermie der chemischen Umwandlung vom tertiären Amin (wie NMM) ins gebildete Aminoxid (wie NMMNO) die Gefahr einer Selbstbeschleunigung, begleitet von einem unkontrollierten Temperatur- und Druckanstieg. Nebenbei wird die Produktqualität in einen unkontrollierten höheren Temperaturbereich negativ beeinflusst. Die Selektivität der Umsetzungsreaktion sinkt und unerwünschte Nebenprodukte können gebildet werden. Solche Nebenprodukte können ausgehend vom tertiären N-Methyl-Morpholin (NMM) beispielsweise Morpholin (M), und Nitrosomorpholin (NMOR) sein. Aus diesen Gründen ist die Reaktionstemperatur im Hohlkörper bzw. des Reaktionsfluids vorzugsweise 20°C bis 70°C, bevorzugt 50°C bis 65°C, am meisten bevorzugt von 60°C bis 65°C. Diese Temperatur kann durch eine entsprechende temperierte Ummantelung der Hohlkörper des Reaktors eingestellt werden.

Ein bevorzugter Druck im Hohlkörper ist 1 bar bis 200 bar, bevorzugt 5 bar bis 100 bar, am meisten bevorzugt 50 bar bis 100 bar. Bei diesen Drücken findet eine rasche Reaktion statt. Des Weiteren lässt sich bei hohen Drücken $CO_2$ besser lösen und somit mit dem Produktfluid vermischen.

Die Hohlkörper können aus diversen Materialien bestehen, z.B. Metallen, Keramik oder Kunststoffen. Die Innenwand des Hohlkörpers kann einen Einfluss auf die Oxidationsreaktion haben. Vorzugsweise enthält die Innenwand eines Hohlkörpers des Reaktors ein oder mehrere Metalle, vorzugsweise ausgewählt aus Fe, Cr, Ni, Mo, Mn, Nb, Au, Pt, Ti oder Mischungen hiervon. Besonders geeignet sind Edelstähle der Klassen 1.43, 1.44 und 1.45, da diese eine sehr hohe Wärmeleitfähigkeit bei gleichzeitig guter Korrosionsbeständigkeit gegenüber stark oxidativen Medien (wie z.B. $H_2O_2$) besitzen. Vorzugsweise enthält eine Innenwand eines Hohlkörpers Austenit-Stahl. Zusätzlich sollte das Hohlkörpermaterial druckbeständig sein - je nach gewünschtem Reaktionsdruck. Diese Kombination von hoher Wärmeleitfähigkeit, hoher Korrosionsbeständigkeit und hoher Druckbeständigkeit verlangt meist Metall oder Keramikmaterialien. Kunststoffe können für Einbauten (z.B. statischen Mischern) oder zusätzliche Beschichtungen verwendet werden. Als Einlagen bzw. Einbauten, welche vor allem zum Zwecke der besseren Durchmischung der Reaktionslösung eingesetzt werden, benötigt es lediglich eine hohe Korrosionsbeständigkeit, wobei hier Materialien, wie die oben genannten, insbesondere auch Eisen, Titan, Gold, Platin, Keramik, Glas, PTFE, PEEK etc. Einsatz finden.

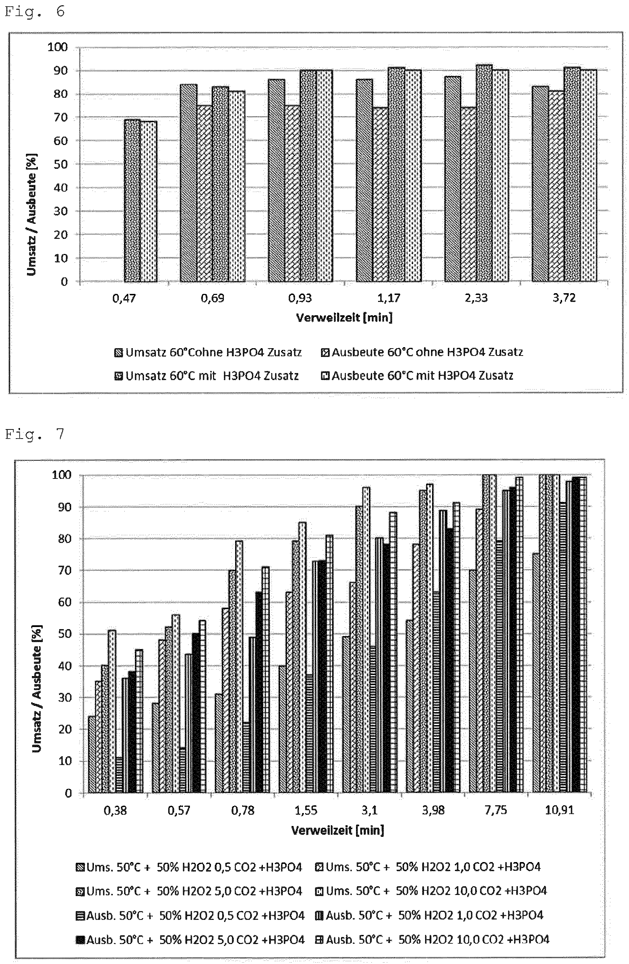

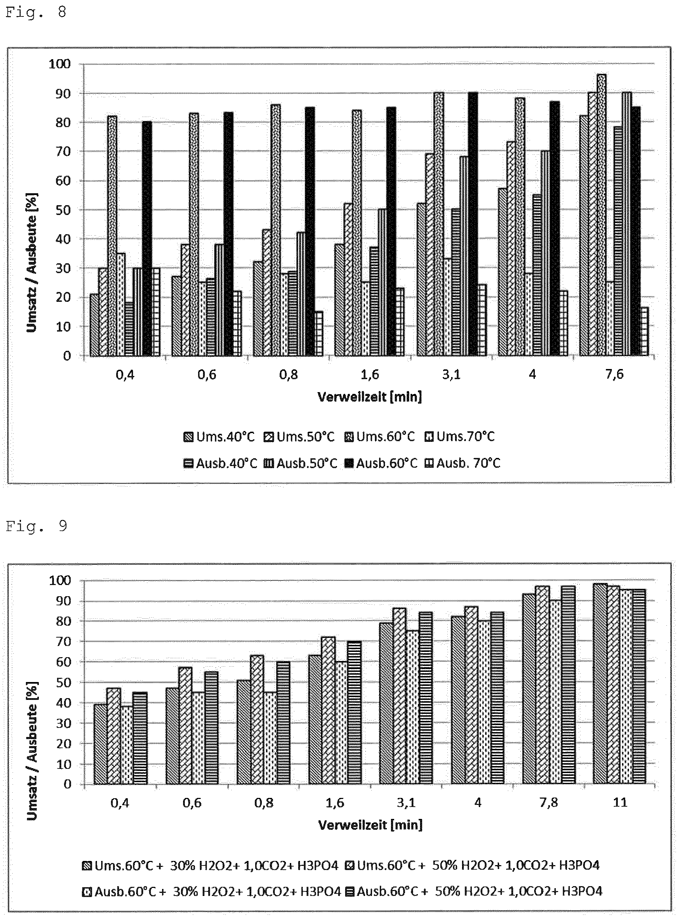

Die Hohlkörper können jede beliebige Form einnehmen, z.B. der Innenquerschnitt kann rund, kreisförmig, quadratisch, halbkreisförmig, hexaedrisch, oktaedrisch usw. sein. Vorzugsweise sind die Hohlkörper Rohre, auch vorzugsweise mit rundem, kreisförmigem, quadratischen, halbkreisförmigen, hexaedrischen, oktaedrischen usw. Innenquerschnitt. Der erfindungsgemäße Reaktor wird hierin auch Rohrreaktor bezeichnet. Zusätzlich sind Einbauten, möglich, welche in beliebig gewählten Abständen oder auch vereinzelt vorgesehen werden. Derartige Einbauten sind beispielsweise Verengungen oder Quetschungen zur Erhöhung der Turbulenz der Strömung, insbesondere zum besseren Vermischen des Fluids im Rohr. Ebenso kann ein oder mehrere statische Mischer (z.B. Mischplättchen) oder Lochplatten vorgesehen werden. Mischzeiten von 1ms bis 1s sind bevorzugt. Geeignete Lochplaten zum Vermischen werden beispielsweise in Fig. 14 der WO 2010/055034 (unter Bezugnahme hierin aufgenommen) gezeigt. Geeignete Quetschungen sind in Fig. 7 und 8 der WO 2010/055034 gezeigt.

Das tertiäre Amin kann in einer Konzentration von 40% bis 100%, vorzugsweise von 50% bis 99,5%, insbesondere 60% bis 98%, speziell bevorzugt 70% bis 97%, in den Reaktor zugeführt werden (alle % in vol.-%). Diese Konzentrationen können auch zu Beginn der Reaktion vorliegen, ggf. verdünnt je nach Volumen eines zugesetzten Fluids mit Oxidationsmittel oder anderer Zusätze.

Vorzugsweise wird ein Oxidationsmittel, insbesondere Wasserstoffperoxid, in einer Konzentration von 5% bis 80%, vorzugsweise von 10% bis 70%, insbesondere 20% bis 60%, speziell bevorzugt 30% bis 55%, in den Hohlkörper zugeführt (alle % in Gew.-%). Die Menge sollte mit dem tertiären Amin abgestimmt werden, sodass die obigen Verhältnisse, insbesondere 1:1 moläquivalent tertiäres Amin zu Oxidationsmittel, insbesondere Peroxid, erfüllt sind.

Das Reaktionsfluid kann bei einer Bodensteinzahl von größer als 10, bevorzugt von 20 bis 200, gemäß der Formel

$$Bo = u * L/D_{ax},$$

worin Bo die Bodensteinzahl ist, u die Strömungsgeschwindigkeit des Reaktionsfluids, L die Länge des Hohlkörpers des Reaktors und $D_{ax}$ der axiale Dispersionsstrom des Reaktionsfluids ist, durch den Reaktor befördert werden. Hierzu kann eine geeignete Strömungsgeschwindigkeit bei gegebener Reaktor/Hohlkörpergeometrie ausgewählt werden. Die Bodensteinzahl beschreibt den Grad der Rückvermischung im Reaktor. Sie ist das Verhältnis zwischen dem Konvektionsstrom (u*L) und dem axialen Dispersionsstrom. Im Bereich der gewählten Bodensteinzahlen ergeben sich für die Einbringung des Amins und des Oxidationsmittels zur Bildung des Reaktionsfluides und in weiterer Folge der Oxidation geeignete Mischzustände.

Vorzugsweise wird $CO_2$ in einer Menge von 0,5 Gew.-% bis 20 Gew.-%, bezogen auf das tertiäre Amin, in das Reaktionsfluid eingebracht. $CO_2$ kann zusammen mit dem Amin oder dem Oxidationsmittel oder separat, vorzugsweise nach vorheriger Sättigung in Lösung oder bei mindestens 90 % der Sättigungskonzentration in der jeweiligen Lösung, eingebracht werden oder direkt in den Mikroreaktor eingespeist werden. Vorzugsweise ist die Lösung von $CO_2$ basisch, z.B. mit pH 8-11 oder 9-10, um die Löslichkeit von $CO_2$ zu erhöhen. Vorzugswese wird $CO_2$ im tertiären Amin, ggf. mit Wasser gelöst, oder direkt in das Reaktionsfluid eingebracht, z.B. fluid unter Druck oder als Lösung in Wasser. $CO_2$ kann die Oxidation von tertiären Aminen zu Aminoxid erheblich beschleunigen, im Fall von NMM zu NMMO um das 460-fache. Mechanistisch kommt es zu einer Reaktion von Peroxid ($RHO_2$, insbesondere $H_2O_2$) mit $CO_2$ und das gebildete Peroxymonocarbonat $HCO_4^-$ oder auch $RCO_4^-$ wirkt beschleunigend auf die Reaktion von NMM zu NMMO bzw. ist selbst das aktivere Oxidationsmittel als die eingesetzte Oxidform (wie $H_2O_2$). Da somit $CO_2$ selbst als Reaktionspartner eingreifen kann, kann eine hohe Konzentration die Reaktion positiv beeinflussen. Bei Versuchen mit dem erfindungsgemäßen Reaktor wurde festgestellte, dass besonders hohe und effektive $CO_2$ Mengen (z.B. wenn vollständig oder nahezu vollständig gelöst) eingesetzt werden können. Vorzugsweise wird $CO_2$ unter Druck und gelöst dem Reaktionsfluid zugeführt. Vorzugsweise sind die oben genannten $CO_2$-Mengen gelöstes $CO_2$. Im erfindungsgemäßen kontinuierlichen System können besonders hohe $CO_2$ Mengen erreicht werden und diese haben positive Auswirkungen auf die Reaktion. Beispielsweise kann die $CO_2$ Menge mindestens 0,5%, mindestens 1%, mindestens 2%, mindestens 4%, mindestens 6%, mindestens 8%, mindestens 10%, mindestens 15%, alle Gew.-%, bezogen auf das tertiäre Amin oder eine andere in das Reaktionsfluid eingebrachte Lösung von $CO_2$, betragen. Ebenfalls sind diese Mengen bezogen auf das gesamte Reaktionsfluid möglich.

Zusätzlich kann ein Metallkomplexbildner, vorzugsweise ein Phosphat, dem Reaktionsfluid zugefügt werden, vorzugsweise in einer Menge von 0,01% bis 3%, vorzugsweise 0,03% bis 2%, speziell bevorzugt 0,05% bis 1,5% (alle Gew.-%). Unerwünschte Metalle im Fluid können das Oxidationsmittel zersetzen und daher die Reaktivität gegenüber dem tertiären Amin senken. Der Metallkomplexbildner soll daher diese Wirkung des Metalls verhindern oder reduzieren. Jeder beliebige Komplexbildner, z.B. Chelatbildner, ist möglich (siehe z.B. einleitend erwähnt). Überraschenderweise wurde festgestellt, dass Phosphat ebenfalls geeignet ist, insbesondere bei eisenhaltigen Reaktormaterialien, welche Eisen abgeben können bzw. deren Eisenmantel selbst an der Oberfläche zum Reaktionsfluid störend einwirken kann. Dies konnte mittels Metallkomplexbildner reduziert werden, sodass etwa 10% höhere Ausbeuten erzielt werden konnten.

Vorzugsweise wird das tertiäre Aminoxid vorgewärmt bevor es oxidiert wird. Das Vorwärmen ist insbesondere auf die im Reaktionsfluid gewünschte Temperatur, z.B. ausgewählt aus den oben genannten Temperaturen. Vorwärmen kann in einem Reaktor oder Hohlkörper wie oben beschrieben vorgenommen werden und kann bei entsprechend hohen Oberflächen zu Volumenverhältnissen relativ rasch erfolgen (z.B. Verhältnisse wie während der oben dargestellten Reaktion). Vorwärmen kann beispielsweise in einem Zeitraum von 5 sec bis 5 min erfolgen, vorzugsweise etwa 1 min. Etwa bedeutet +/- 20%.

Ebenso kann ein Oxidationsmittel vorgewärmt werden. Hierzu können ebenso die für das tertiäre Amin beschriebenen Maßnahmen und Parameter gewählt werden, z.B. Vorwärmen in einem Zeitraum von 5 sec bis 5 min, vorzugsweise etwa 1 min.

Nach erwünschtem Reaktionsfortschritt, z.B. wenn das gesamte tertiäre Amin oder beinahe das gesamte tertiäre Amin (z.B. 99,5% oder mehr) oxidiert wurde, kann verbleibendes Oxidationsmittel abgebaut oder entfernt werden. Im Fall von Peroxiden, insbesondere Wasserstoffperoxid, eignet sich der Einsatz von Katalase oder die Behandlung mit einem anderen Katalysator wie $MnO_2$ oder Platin (z.B. platinierter Nickel). Der Katalysator kann in Form von Bündeln oder Partikeln inhärent mit hoher Oberfläche (z.B. Katapak®) oder als Katalysatorblech eingesetzt werden.

Anstatt sauberen tertiären Aminen können auch aufkonzentrierte Brüden, welche bei der Herstellung von Cellulosefasern anfallen, verwendet werden. Die Aufkonzentrierung wurde wie in EP 0 448 924 B1 beschrieben durch Umkehrosmose durchgeführt. Morpholin wurde, wie im EP 0 448 924 B1 beschrieben, in einem abschließenden Schritt abdestilliert und kann nach einer Methylierung, wie beispielsweise im Patent DE 3504899 A1 beschrieben, ebenfalls oxidiert werden.

Das erfindungsgemäß produzierte Aminoxid, vorzugsweise NMMO, kann direkt eingesetzt werden. Das erfindungsgemäße kontinuierliche Verfahren eignet sich hier besonders das laufend produzierte Aminoxid in eine ebenfalls kontinuierlich betriebene Folgeanlage einzusetzen. Ein solcher gekoppelter Folgeprozess ist beispielsweise das Auflösen von Zellulose in NMMO oder anderen Aminoxiden. Wie einleitend erwähnt kann eine Zelluloselösung in Aminoxid für ein Spinnverfahren (Lyocellverfahren) eingesetzt werden. Hierbei können aus der Zelluloselösung Filamente, Fasern oder Folien geformt und hergestellt werden. Auch dieses ist mit dem erfindungsgemäßen Verfahren in-line oder off-line kombinierbar, analog wie der Auflösungsvorgang.

Gegebenenfalls kann das erfindungsgemäß produzierte Amninoxid aufkonzentriert oder verdünnt werden, vorzugsweise nach einem Abbau oder der Entfernung des Oxidationsmittels. Vorzugsweise wird die Konzentration des erhaltenen Aminoxid auf 20%-60%, insbesondere bevorzugt auf 30%-50% eingestellt (alle Gew.-%). Derartige Zusammensetzungen eignen sich gut zur Lagerung oder zum Transport (inklusive zum Transport durch Rohrleitungen in einer gekoppelten Anlage). Zum Auflösen von Zellulose werden vorzugsweise höhere Konzentrationen eingestellt. So kann das produzierte Aminoxid auf eine Konzentration von 50% bis 95%, vorzugsweise 60% bis 90% oder 70% bis 80% eingestellt werden (alle Gew.-%). Als Verdünnungsmittel kann Wasser oder jede andere mischbare Flüssigkeit eingesetzt werden.

Beim Auflösen und/oder zum Spinnen von Zellulose im produzierten Aminoxid wird vorzugsweise eine Lösung mit 4% bis 30%, vorzugsweise 8% bis 25% oder 10% bis 20%, Zellulose erzeugt (alle Gew.-%). Eine geeignete Spinnlösung hat zum Beispiel die Zusammensetzung Zellulose: 12,9%, NMMO 76,3%, Wasser 10,8%, alle % in Gew.-% (siehe WO 2013/030400, durch Bezugnahme hierin aufgenommen). In der WO 2013/030400 wird weiters ein Spinnverfahren beschrieben, welche erfindungsgemäß eingesetzt werden kann. Im Allgemeinen kann die Zelluloselösung mit dem erfindungsgemäß produzierten Aminoxid extrudiert werden, wobei die Extrudate einen Gasspalt passieren und in ein Fällbad eingebracht werden. Das Fällbad kann ein Fällmittel, z.B. Wasser, in einer Konzentration zum Ausfällen der Zellulose aufweisen. Im Gasspalt kann ein Gasstrom in an sich bekannter Weise geführt werden.

Demgemäß umfasst die vorliegende Erfindung auch ein Verfahren, wobei das erfindungsgemäß produzierte Aminoxid auf eine Konzentration zum Auflösen von Zellulose gebracht wird und optional weiters die Zellulose darin aufgelöst wird und optional weiters die aufgelöste Zellulose in Endlosformkörper, z.B. Filamente, Fäden oder Folien, geformt wird, z.B. durch Extrusion, und anschließend die Zellulose der Formkörper gefällt wird.

Reaktorsystem:

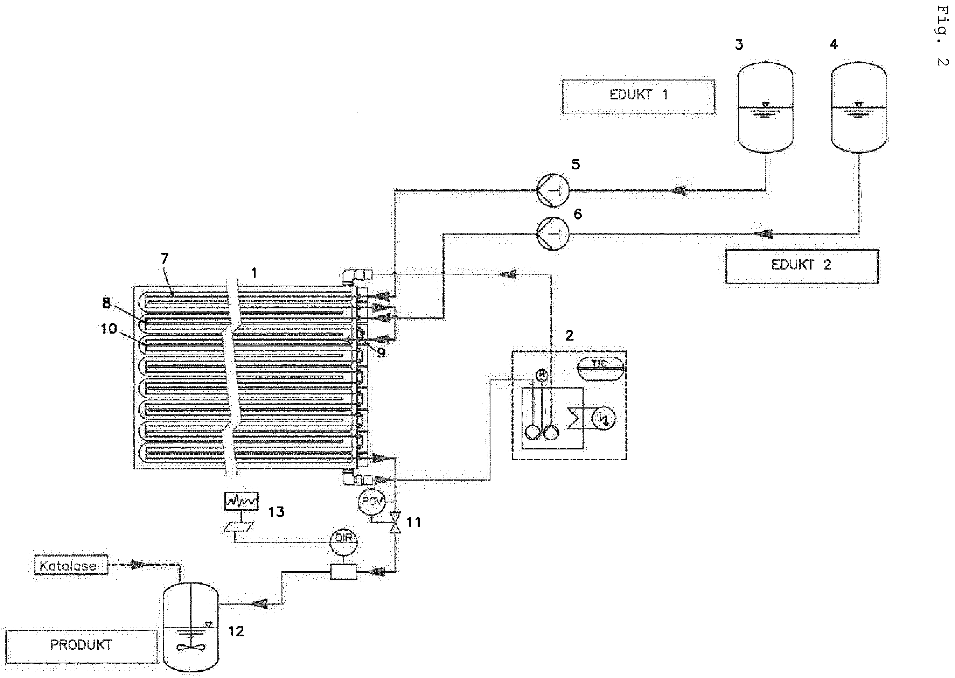

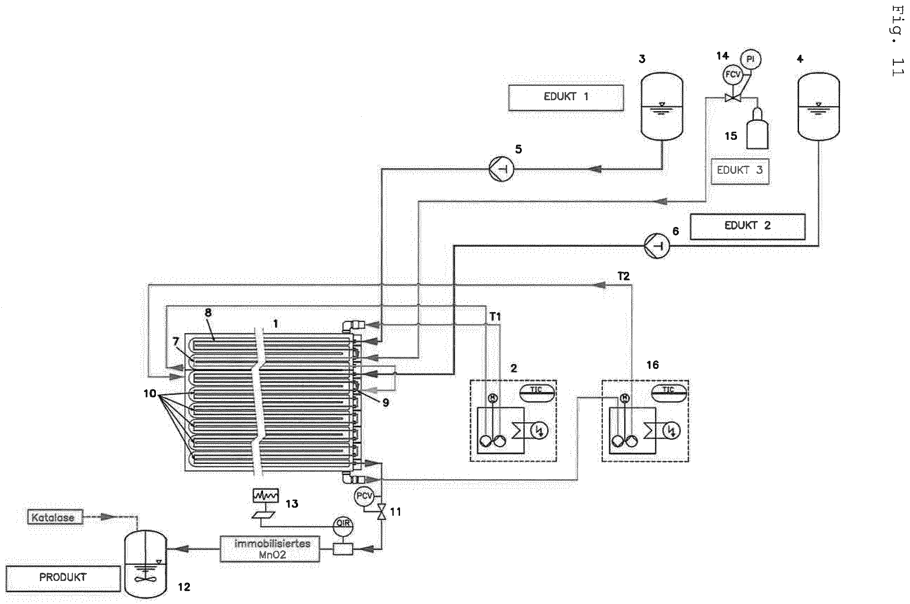

Ein erfindungsgemäßer Reaktor in einem Reaktorsystem ist in Figur 2 und 11 dargestellt. Es wurde ein Mikroreaktor (1) wie in WO 2010/055034 beschrieben eingesetzt. Das Reaktorsystem hat einen Rohrreaktor (1), der zur Führung des Reaktionsfluids verwendet wird, sowie zum Vorheizen der beiden Edukte (tertiäres Amin und Oxidationsmittel). Die Edukte werden aus Vorlagebehältern (3, 4) über Pumpen (5, 6) zum Reaktor (1) geführt. Dort werden in Zonen 7 und 8 die Edukte vorgewärmt. Zum Beheizen wird ein Temperiermedium um die Hohlkörper geführt. Das Temperiermedium wird im Thermostat (2) geheizt oder gekühlt. Die vorgewärmten Edukte werden an der Position 9 zusammengeführt, wodurch das Reaktionsfluid entsteht und durch die Hohlkörper, z.B. Reaktorrohre 10 geführt wird. Druck wird sowohl über die Pumpen (5, 6) als auch über ein Druckhalteventil (11) gesteuert. Das Produkt wird in einem Behälter (12) aufgefangen, welcher über ein Rührwerk verfügt. Hier können optional noch weitere Reaktionen durchgeführt werden, z.B. Durchoxidieren von verbliebenen Amin oder Abbau von Oxidationsmittel. Ein Messgerät mit Durchflusszelle (13) kann optional den Produktabfluss überwachen.

Die vorliegende Erfindung wird weiter durch die folgenden Beispiele erläutert und eingehender beschrieben, ohne auf diese Ausführungsformen der Erfindung beschränkt zu sein. Sämtliche Parameter und Apparatteile dieser Beispiele sind auch isoliert von der restlichen Beispielsbeschreibung mit den oben genannten Hauptaspekten der Erfindung oder den bevorzugten Ausführungsformen kombinierbar.

Beispiele:

Chemikalien:

Die zur Durchführung der Experimente eingesetzten Chemikalien wurden in verschiedener Reinheit von unterschiedlichen Lieferanten bezogen. Tabelle 1 zeigt eine Aufstellung der für die Versuchsdurchführung benutzten Chemikalien.

Tabelle 1: Chemikalien

| Bezeichnung | Lieferant | Reinheit [%] | Katalognummer | CAS-Nummer |
|---|---|---|---|---|
| **NMM** | Alfa-Aesar | > 99 | A12158 | 109-02-4 |
| **$H_2O_2$ 30%ig** | Sigma | - | 216763 | 7722-84-1 |
| **$H_2O_2$ 50%ig** | Sigma | - | 516813 | 7722-84-1 |
| **Katalase (lagern bei - 20°C)** | Sigma | 2,000 - 5,000 units/mg | C-9322 | 9001-05-2 |
| **$CO_2$ N45** | Air Liquide | 99,995% | | |
| **Kaliumpermanganat** | Sigma | 0,2M | 35184 | 7722-64-7 |
| **Phosphorsäure** | Carl Roth | 75%, reinst. | 2614.1 | 7664-38-2 |
| **Schwefelsäure Kühlmittel Wasser/ Ethylenglykol 1/1 Vol./Vol.** | Bernd Kraft | 2,5M | 03279.3000 | |

| **HPLC-UV** | | | | |
|---|---|---|---|---|
| Bezeichnung | Lieferant | Reinheit [%] | Katalognummer | CAS-Nummer |
| **Morpholin** | Sigma | ≥ 99 | 252360 | 110-91-8 |
| **NMMNO x $H_2O$** | Alfa-Aesar | 98+ | A15996 | 70187-32-5 |
| **NMMNO 50 Gew.% in $H_2O$** | Alfa-Aesar | - | A19802 | 7529-22-8 |

**Reaktionssystem nach Fig. 2**

Lösen von $CO_2$ in N-Methylmorpholin

Zur Erhöhung der Reaktionsgeschwindigkeit wird Edukt 1 mit [F] Gew.-% $CO_2$ gesättigt. Dies passierte z.B. durch das Eintragen von $CO_2$ über ein Gaseinleitungsrohr oder eine gesinterte, poröse Vorrichtung in die NMM-Lösung. NMM wurde auch mit einer $CO_2$-Atmosphäre überlagert um unter Rühren bei 0,1-20 bar Überdruck $CO_2$ in NMM zu lösen. Nach abgeschlossener Dosierung wurde die jeweilige $CO_2$ angereicherte Eduktlösung zur Kontrolle abgewogen und für die Mikroreaktorversuche eingesetzt.

Reaktionsführung im Mikroreaktor

Das von uns verwendete Mikroreaktionssystem basiert auf der WO 2010/055034 und wurde aus dem Werkstoff [A] gemäß Tabelle hergestellt. Die Mikroreaktorversuche der Synthese von N-Methylmorpholin-N-oxid (NMMNO) aus 4-N-Methyl-Morpholin (NMM) und Wasserstoffperoxid ($H_2O_2$) wurden in unterschiedlichen Aufbauten im Mikroreaktor durchgeführt. Die Kenndaten des eingesetzten Reaktors (1) waren wie folgt:

Volumen: variabel, bis zu 26 mL
Kapillardurchmesser: 1 mm, Rohre mit Quetschungen

Da die Umsetzung von NMM mit $H_2O_2$ zu NMMNO exotherm (adiabater Temperaturanstieg 335 K) erfolgt, ist bei dieser Reaktion sicherzustellen, dass die Mikroreaktorbedingungen und technische Auslegung so gewählt sind, dass die Bedingungen für den Wärmetransport ausreichend geeignet sind, sodass dem Reaktionssystem einerseits dem Edukt NMM Wärme zugeführt, andererseits aus der Reaktionslösung Wärme abgeführt werden kann. Dies geschieht durch ausreichend dimensionierte Mikroreaktoroberflächen.

Dank der großen Austauschfläche zwischen Reaktionsmedium und Temperiermedium kann die Wärme kontrolliert abgeführt werden und es erübrigen sich lange Dosierzeiten, welche im Batchverfahren bei mehreren Stunden liegen. Des Weitern befinden sich im Mikroreaktor kleinere Reaktionsvolumina, wodurch die Sicherheit des Verfahrens erheblich gesteigert wird. Durch die verbesserte Prozesskontrolle können eine verbesserte Qualität, Selektivität und eine höhere Ausbeute verzeichnet werden. Des weiteren ermöglicht der Versuchsaufbau das Arbeiten unter erhöhtem Druck, wodurch sich der Stofftransport/Phasentransfer im Vergleich zum Batchreaktor stark verbessert.

Es wurde ein Heizthermostat (2) ECO SILVER RE415 der Firma Lauda eingesetzt um den Mikroreaktor auf [B] °C zu temperieren. Als Kühl- und Heizmedium wurde ein Gemisch aus Wasser/ Ethylenglycol im Volumensverhältnis 1:1 eingesetzt.

In zwei Vorratsbehältern (3, 4) werden die beiden Edukte vorgelagert.

Tabelle 2: Edukte

|  | Edukt 1 | Edukt 2 |
|---|---|---|
| Volumenstrom | [C] ml/min | [D] ml/min |
| Molares Verhältnis | | [E] |
| Zusammensetzung | 8,5 M N-Metyhlmorpholin vorgesättigt mit [F] Gew.% $CO_2$ | [G] Gew.-%iges Wasserstoffperoxid mit [H] gelöstem $CO_2$ und [I] Gew.% $H_3PO_4$ |

Über zwei computergesteuerte Pumpen (5 und 6) des Typs SyrDos von HiTec Zang werden die Volumenströme der Chemikalien geregelt und nach den Vorwärmrohren (7 und 8) über eine Mischmodul T3 mit eingesetztem Statischen Mischer (Z.B. Mischplättchen) (9), gemäß WO 2010/055034, zusammengeführt. Im Mischmodul können unterschiedliche Mischeinsätze eingesetzt werden. Anschließend wird das Reaktionsgemisch in die Reaktionsrohre (10) geleitet, wo dann die Reaktion stattfindet. Zur Erhöhung und Regulierung des Drucks im Reaktor wurde vor der Durchflusszelle ein Druckhalteventil (11) für den Bereich 0-20 bar eingesetzt und auf [J] bar geregelt. Ein weiteres Druckhalteventil kann nach der Durchflusszelle installiert werden um Gasbildung in der Zelle zu vermeiden. Der Einbau des Druckhalteventils sollte die Zerfalls- und Nebenreaktionen möglichst unterdrücken, da es im Zuge dieser unerwünschten Reaktionen zur Gasentwicklung kommt.

Das gewünschte Produkt wird in einem Gefäß mit Rührwerk (12) aufgefangen. Überschüssiges Wasserstoffperoxid wird durch langsames Zutropfen von 0,5 Gew.-%iger Katalaselösung zersetzt und anschließend einem Peroxidtest unterzogen.

Durchsätze von [K] ml/min mit Umsätzen von [L] % und Ausbeuten von [M] % konnten erreicht werden.

Tabelle 3: Verfahrenstechnische Parameter

| | |
|---|---|
| Fluidgeschwindigkeit | [N] m/min |
| Hydrodynamische Verweilzeit im Reaktionsbereich | [O] min |
| Reaktorspezifische Oberflächenbelastung | [P] l/$m^2$h |
| Reaktorspezifische Volumensbelastung | [Q] l/$m^3$h |
| Oberflächen:Volumen Verhältnis | [R] |

Analytik

Über eine online FT-IR Messung (13) in einer Durchflusszelle mit dem Spektrometer Alpha von BRUKER wird die Qualität des Produkts aufgezeichnet. Der Peak bei 1045-1028 cm$^{-1}$ welcher dem Edukt zugeordnet werden kann wird zur Verfolgung des Umsatzes verwendet. Die Peaks, die dem NMMNO zugeordnet werden können, erscheinen bei den Wellenzahlen 1250-1215 cm$^{-1}$, 995-960 cm$^{-1}$ und 960-920 cm$^{-1}$. Zur Auswertung der Ausbeute wird der Peak

bei 1250-1215cm$^{-1}$ verwendet.

Die Reaktionsproben wurden ebenfalls off-line mittels HPLC/UV analysiert. Es werden die Proben mittels Flüssigchromatographie getrennt und über einen UV-Detektor gemessen. Anhand der erhaltenen Chromatogramme werden die Versuche ausgewertet. Zur Analyse wurde ein HPLC 1100 der Firma Agilent Technologies verwendet.

Die erhaltenen Chromatogramme aus der Kalibrierung zeigten NMMNO bei einer Retentionszeit von 1,940 Minuten, sowie NMM mit einer Retentionszeit von 17,341 Minuten und Morpholin mit einer Retentionszeit von 5,461 Minuten an. Das Chromatogramm der Einspritzung eines sythetische hergestellten Referenzgemisches (NMM, NMMNO, M) zeigte, dass die Substanzen sauber voneinander getrennt werden und bestimmt werden können.

Zur Bestimmung von gegebenenfalls gebildetem N-Nitrosomorpholin (NMOR) wurde, auf Grund der zu erreichenden Konzentrationen von < 100 ppb, ein Gaschromatograph mit Massenspektrometrie-Kopplung verwendet, da in diesen Bereichen die Genauigkeit von HPLC/UV Systemen nicht ausreicht. NMOR kann durch Einsatz mittelpolarer Lösungsmittel aus dem Produkt extrahiert werden. Bei der Probenvorbereitung hat sich die Verwendung einer 1:1 Mischung von Dichlormethan (DCM) und Ameisensäureethylester (EF) als zweckmäßig erwiesen.

Das Produkt wurde ebenfalls refraktometrisch bei Brechungsindexen von 1,330 - 1,449 bestimmt. Anhand des Brechungsindex kann die Konzentration des synthetisierten NMMNOs abgelesen werden. Die Konzentration des Produkts wurde auch über die Dichte bestimmt.

CO$_2$-Beimischung

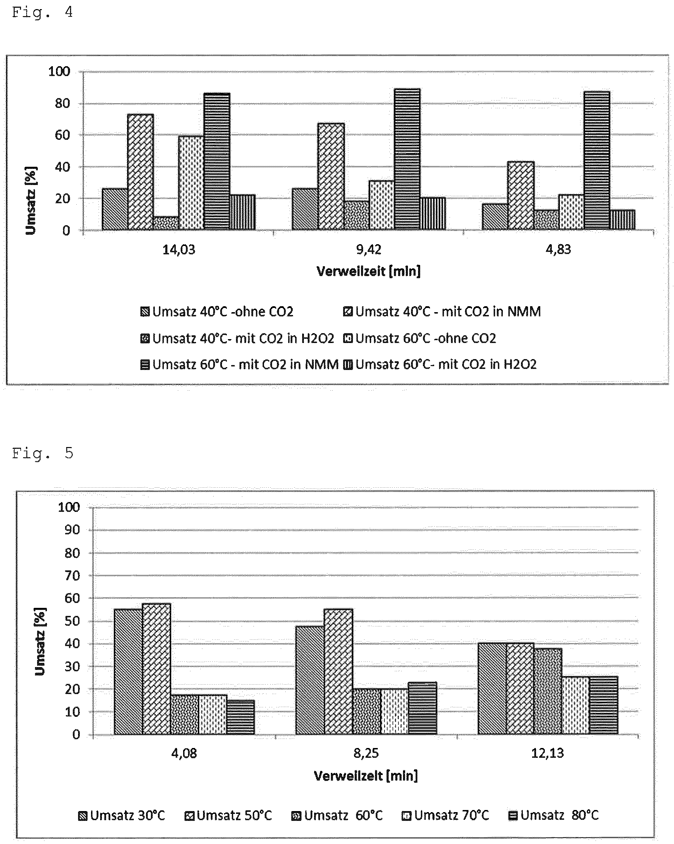

Es wurden Versuche durchgeführt, die zeigen sollten, worin das CO$_2$ für möglichst hohe Umsätze und Ausbeuten gelöst werden soll. Es zeigte sich, dass die Zugabe von CO$_2$ zum NMM sich in allen Fällen positiv auf die Ausbeute auswirkt (+40-70%, Fig. 3 und 4). Der Grund dafür ist in der hohen Löslichkeit von CO$_2$ im

N-Methyl-Morpholin zu finden.

Bei kürzeren Verweilzeiten hat eine erhöhte Temperatur größere Auswirkung auf die Ausbeute als bei längeren Verweilzeiten. Die Reaktion läuft bei erhöhten Temperaturen also schneller ab, was die Produktionsrate durch die möglichen kürzeren Verweilzeiten steigert.

Stabilisierung des Wasserstoffperoxids mit Phosphorsäure zur Reaktionsverbesserung

Da sich bei erhöhten Temperaturen immer etwas Wasserstoffperoxid zersetzt und dadurch der Reaktionslösung fehlt wurde versucht, dies durch Phosphorsäure zu unterdrücken. Es konnte festgestellt werden, dass die Umsätze und Ausbeuten durch Zugabe von H$_3$PO$_4$ um 5-10% erhöht werden (Fig. 6). Dieser Effekt verstärkt sich mit einer Erhöhung der Verweilzeit. Phosphorsäure wirkt als Komplexbildner, welcher katalytisch wirkende MetallIonen, welche das Wasserstoffperoxid zersetzen könnten, komplexiert.

Tabelle 4: Parameter zu Versuchen der $CO_2$-Beimischung

| A Werkstoff | B Temperatur [°C] | C Edukt 1 N-Methyl-Morpholin (NMM) [ml/min] | D Edukt 2 Wasserstoffperoxid (H2O2) [ml/min] | E Molverhältnis Edukt 1 : Edukt 2 [mol/mol] | F CO2 in NMM [Masse %] | G Konzentration H2O2 [Masse %] | H CO2 in H2O2 [Masse %] | I H3PO4 in H2O2 [Masse %] | J Druck [bar] | K Durchsatz [ml/min] | L Umsatz [%] | M Ausbeute NMMO [%] | N Fluidgeschwindigkeit [m/min] | O hydrodynamische Verwellzeit Im Reaktionsbereich -Reaktorlänge [min] | P Reaktor spezifische Oberflächen belastung [l/m²h] | Q Reaktor-spezifische Volumens belastung [l/m³h] | R Oberfläche:Volumen Verhältnis |
|---|---|---|---|---|---|---|---|---|---|---|---|---|---|---|---|---|---|
| PTFE | 40 | 0,23 | 0,2 | 1:1 | 0 | 29 | 0 | 0 | 6,89 | 0,43 | 26 | 25 | 0,21 | 14,03 | 1,71 | 4.277,42 | 2,5 |
| | 40 | 0,34 | 0,3 | 1:1 | 0 | 29 | 0 | 0 | 6,89 | 0,64 | 26 | 26 | 0,32 | 9,42 | 2,55 | 6.366,39 | 2,5 |
| | 40 | 0,67 | 0,58 | 1:1 | 0 | 29 | 0 | 0 | 6,89 | 1,25 | 16 | 15 | 0,62 | 4,83 | 4,97 | 12.434,35 | 2,5 |
| | 40 | 0,23 | 0,2 | 1:1 | 0,5 | 29 | 0 | 0 | 6,89 | 0,43 | 73 | 70 | 0,21 | 14,03 | 1,71 | 4.277,42 | 2,5 |
| | 40 | 0,34 | 0,3 | 1:1 | 0,5 | 29 | 0 | 0 | 6,89 | 0,64 | 67 | 65 | 0,32 | 9,42 | 2,55 | 6.366,39 | 2,5 |
| | 40 | 0,67 | 0,58 | 1:1 | 0,5 | 29 | 0 | 0 | 6,89 | 1,25 | 43 | 41 | 0,62 | 4,83 | 4,97 | 12.434,35 | 2,5 |
| | 40 | 0,23 | 0,2 | 1:1 | 0 | 29 | zugef. | 0 | 6,89 | 0,43 | 8 | 5 | 0,21 | 14,03 | 1,71 | 4.277,42 | 2,5 |
| | 40 | 0,34 | 0,3 | 1:1 | 0 | 29 | zugef. | 0 | 6,89 | 0,64 | 18 | 15 | 0,32 | 9,42 | 2,55 | 6.366,39 | 2,5 |
| | 40 | 0,67 | 0,58 | 1:1 | 0 | 29 | zugef. | 0 | 6,89 | 1,25 | 12 | 10 | 0,62 | 4,83 | 4,97 | 12.434,35 | 2,5 |
| | 60 | 0,23 | 0,2 | 1:1 | 0 | 29 | 0 | 0 | 6,89 | 0,43 | 59 | 55 | 0,21 | 14,03 | 1,71 | 4.277,42 | 2,5 |
| | 60 | 0,34 | 0,3 | 1:1 | 0 | 29 | 0 | 0 | 6,89 | 0,64 | 31 | 30 | 0,32 | 9,42 | 2,55 | 6.366,39 | 2,5 |
| | 60 | 0,67 | 0,58 | 1:1 | 0 | 29 | 0 | 0 | 6,89 | 1,25 | 22 | 18 | 0,62 | 4,83 | 4,97 | 12.434,35 | 2,5 |
| | 60 | 0,23 | 0,2 | 1:1 | 0,5 | 29 | 0 | 0 | 6,89 | 0,43 | 86 | 85 | 0,21 | 14,03 | 1,71 | 4.277,42 | 2,5 |
| | 60 | 0,34 | 0,3 | 1:1 | 0,5 | 29 | 0 | 0 | 6,89 | 0,64 | 89 | 85 | 0,32 | 9,42 | 2,55 | 6.366,39 | 2,5 |
| | 60 | 0,67 | 0,58 | 1:1 | 0,5 | 29 | 0 | 0 | 6,89 | 1,25 | 87 | 86 | 0,62 | 4,83 | 4,97 | 12.434,35 | 2,5 |
| | 60 | 0,23 | 0,2 | 1:1 | 0 | 29 | zugef. | 0 | 6,89 | 0,43 | 22 | 20 | 0,21 | 14,03 | 1,71 | 4.277,42 | 2,5 |
| | 60 | 0,34 | 0,3 | 1:1 | 0 | 29 | zugef. | 0 | 6,89 | 0,64 | 20 | 18 | 0,32 | 9,42 | 2,55 | 6.366,39 | 2,5 |
| | 60 | 0,67 | 0,58 | 1:1 | 0 | 29 | zugef. | 0 | 6,89 | 1,25 | 12 | 10 | 0,62 | 4,83 | 4,97 | 12.434,35 | 2,5 |

Reaktormaterialien

Die Synthese von NMMNO wurde mit unterschiedlichen Kapillarmaterialien durchgeführt. Auch der hochkorrosionsbeständige Werkstoff Hastelloy® C-22 wurde getestet. Hastelloy Legierungen zählen zur Gruppe der hochkorrosionsbeständigen Nickel-Molybdän Legierungen und sind gekennzeichnet durch hohe Nickel, Molybdän und Chromghalte. Diese Werkstoffe zeichnen sich durch hohe Beständigkeiten in reduzierenden Medien aus.

Wie in Fig. 5 erkenntlich, wirkt sich eine Erhöhung der Temperatur bis 50°C-60°C gut auf den Umsatz von NMM aus. Diese Versuche wurden ohne $CO_2$ durchgeführt. Bei weiterer Erhöhung zersetzt sich das Wasserstoffperoxid an der Reaktorwand bevor es mit NMM reagieren kann. Das Reaktionsprodukt wird braun-rot und es herrscht massive Gasentwicklung. Hastelloy® C-22 eignet sich ohne Metallmaskierer eher schlecht für diese Reaktion als Reaktormaterial. Da die Reaktion in Edelstählen der Klasse 1.43, 1.44 und 1.45 problemlos abläuft ist ein möglicher Grund dafür der hohe Anteil von Molybdän und/oder Nickel, der die Zersetzung von Wasserstoffperoxid katalysiert. Vorzugsweise wird der Werkstoff Hastelloy® C-22 erfindungsgemäß nicht eingesetzt oder maskiert.

Nichtrostende, korrosionsbeständigen Stähle austenitische Stähle der Stahlgruppennummern 1.40xx bis 1.45xx sind bevorzugte Kapillar-Rohrmaterialen.

Edelmetalle wie Gold/Platin oder die Übergangsmetalle Titan/Tantal und verschiedenste Polymere wie PEEK, PTFE, PFA, PVDF, PMMA, PVC, PET eignen sich ebenfalls hervorragend als Reaktormaterialien, wie Hohlkörper, insbesondere Kapillaren, Rohre und Mischplättchen als Einzelwerkstoff oder in Kombinationen für die Durchführung dieser Reaktion. Verwendete Hohlkörper-, Rohr- und Kapillarmaterialien können auch beschichtet oder emailliert zum Einsatz kommen.

Tabelle 5: Parameter zu Versuchen der Phosphorsäure Beimischung

| A | B | C | D | E | F | G | H | I | J | K | L | N | O | P | Q | R |
|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoff peroxid (H2O2) [ml/min] | Molverhältnis Edukt 1 : Edukt 2 [mol/mol] | CO2 in NMM [Masse %] | Konzentration H2O2 [Masse %] | CO2 In H2O2 [Masse %] | H3PO4 In H2O2 [Masse % ] | Druck [bar] | Durchsatz [ml/min] | Umsatz [%] | Fluidgeschwindigkeit [m/min] | hydrodynamische Verweilzeit Im Reaktionsbereich - Reaktorlänge [min] | Reaktorspezitische Oberflächen belastung [l/m²h] | Reaktorspezitische Volumens belastung [l/m³h] | Oberfläche: Volumen Verhältnis |
| Hastelloy® C-22 | 30 | 0,18 | 0,16 | 1:1 | 0 | 32 | 0 | 0 | 6,89 | 0,34 | 40 | 0,43 | 12,13 | 1,24 | 4.947,59 | 4 |
| | 30 | 0,27 | 0,23 | 1:1 | 0 | 32 | 0 | 0 | 6,89 | 0,5 | 48 | 0,64 | 8,25 | 1,82 | 7.275,87 | 4 |
| | 30 | 0,54 | 0,47 | 1:1 | 0 | 32 | 0 | 0 | 6,89 | 1,01 | 55 | 1,29 | 4,08 | 3,67 | 14.697,26 | 4 |
| | 50 | 0,18 | 0,16 | 1:1 | 0 | 32 | 0 | 0 | 6,89 | 0,34 | 40 | 0,43 | 12,13 | 1,24 | 4.947,59 | 4 |
| | 50 | 0,27 | 0,23 | 1:1 | 0 | 32 | 0 | 0 | 6,89 | 0,5 | 55 | 0,64 | 8,25 | 1,82 | 7.275,87 | 4 |
| | 50 | 0,54 | 0,47 | 1:1 | 0 | 32 | 0 | 0 | 6,89 | 1,01 | 58 | 1,29 | 4,08 | 3,67 | 14.697,26 | 4 |
| | 60 | 0,18 | 0,16 | 1:1 | 0 | 29 | 0 | 0 | 6,89 | 0,34 | 38 | 0,43 | 12,13 | 1,24 | 4.947,59 | 4 |
| | 60 | 0,27 | 0,23 | 1:1 | 0 | 29 | 0 | 0 | 6,89 | 0,5 | 20 | 0,64 | 8,25 | 1,82 | 7.275,87 | 4 |
| | 60 | 0,54 | 0,47 | 1:1 | 0 | 29 | 0 | 0 | 6,89 | 1,01 | 18 | 1,29 | 4,08 | 3,67 | 14.697,26 | 4 |
| | 70 | 0,18 | 0,16 | 1:1 | 0 | 29 | 0 | 0 | 6,89 | 0,34 | 25 | 0,43 | 12,13 | 1,24 | 4.947,59 | 4 |
| | 70 | 0,27 | 0,23 | 1:1 | 0 | 29 | 0 | 0 | 6,89 | 0,5 | 20 | 0,64 | 8,25 | 1,82 | 7.275,87 | 4 |
| | 70 | 0,54 | 0,47 | 1:1 | 0 | 29 | 0 | 0 | 6,89 | 1,01 | 18 | 1,29 | 4,08 | 3,67 | 14.697,26 | 4 |
| | 80 | 0,18 | 0,16 | 1:1 | 0 | 29 | 0 | 0 | 6,89 | 0,34 | 25 | 0,43 | 12,13 | 1,24 | 4.947,59 | 4 |
| | 80 | 0,27 | 0,23 | 1:1 | 0 | 29 | 0 | 0 | 6,89 | 0,5 | 23 | 0,64 | 8,25 | 1,82 | 7.275,87 | 4 |
| | 80 | 0,54 | 0,47 | 1:1 | 0 | 29 | 0 | 0 | 6,89 | 1,01 | 15 | 1,29 | 4,08 | 3,67 | 14.697,26 | 4 |

Tabelle 6: Parameter zur $CO_2$-Beigabe

| A | B | C | D | E | F | G | H | I | J | K | L | M | N | O | P | Q | R |
|---|---|---|---|---|---|---|---|---|---|---|---|---|---|---|---|---|---|
| Werkstoff | Temperatur [°C] | Edukt 1 N-Methyl-Morpholin (NMM) [ml/min] | Edukt 2 Wasserstoff peroxid (H2O2) [ml/min] | Molverhältnis Edukt 1 : Edukt 2 [mol/mol] | CO2 In NMM [Masse %] | Konzentration H2O2 [Masse %] | CO2 In H2O2 [Masse %] | H3PO4 In H2O2 [Masse % ] | Druck [bar] | Durchsatz [ml/min] | Umsatz [%] | Ausbeute NMMO [%] | Fluidgeschwindigkeit [m/min] | hydrodynamische Verwellzelt Im Reaktionsbereich - Reaktorlänge [min] | Reaktorspezifische Oberflächen belastung [l/m²h] | Reaktor - spezitische Volumens belastung [l/m²h] | Oberfläche Volumen Verhältnis |
| 1.4404 | 60 | 2,7 | 2,42 | 1:1,1 | 0,5 | 29 | 0 | 0 | 20 | 5,12 | 84 | 75 | 6,52 | 0,69 | 21,73 | 86.922,38 | 4 |
| | 60 | 2 | 1,79 | 1:1,1 | 0,5 | 29 | 0 | 0 | 20 | 3,79 | 86 | 75 | 4,83 | 0,93 | 16,09 | 64.342,94 | 4 |
| | 60 | 1,6 | 1,43 | 1:1,1 | 0,5 | 29 | 0 | 0 | 20 | 3,03 | 86 | 74 | 3,86 | 1,17 | 12,86 | 51.440,39 | 4 |
| | 60 | 0,8 | 0,72 | 1:1,1 | 0,5 | 29 | 0 | 0 | 20 | 1,52 | 87 | 74 | 1,94 | 2,33 | 6,45 | 25.805,08 | 4 |
| | 60 | 0,5 | 0,45 | 1:1,1 | 0,5 | 32 | 0 | 0 | 20 | 0,95 | 83 | 81 | 1,21 | 3,72 | 4,03 | 16.128.18 | 4 |
| | 60 | 4 | 3,58 | 1:1,1 | 0,5 | 32 | 0 | 0,1 | 20 | 7,58 | 69 | 68 | 9,65 | 0,47 | 32,17 | 128.685,87 | 4 |
| | 60 | 2,7 | 2,42 | 1:1,1 | 0,5 | 29 | 0 | 0,1 | 20 | 5,12 | 83 | 81 | 6,52 | 0,69 | 21,73 | 86.922,38 | 4 |
| | 60 | 2 | 1,79 | 1:1,1 | 0,5 | 29 | 0 | 0,1 | 20 | 3,79 | 90 | 90 | 4,83 | 0,93 | 16,09 | 64.342,94 | 4 |
| | 60 | 0,8 | 0,72 | 1:1,1 | 0,5 | 29 | 0 | 0,1 | 20 | 1,52 | 92 | 90 | 1,94 | 2,33 | 6,45 | 25.805,08 | 4 |
| | 60 | 0,5 | 0,45 | 1:1,1 | 0,5 | 29 | 0 | 0,1 | 20 | 0,95 | 91 | 90 | 1,21 | 3,72 | 4,03 | 16.128,18 | 4 |

Einfluss der $CO_2$ Konzentration auf die Reaktionsgeschwindigkeit

Der positive Effekt der Zugabe von $CO_2$ zur Reaktionslösung wird genauer untersucht. Um ihn zu quantifizieren, wurden Versuche bei unterschiedlichen Gew.% $CO_2$ (gelöst und bezogen auf das Amin) durchgeführt. Wie in Fig. 7 ersichtlich ist, steigt die Reaktionsgeschwindigkeit mit den gelösten Gew.% deutlich an. Parameter und Ergebnisse sind in Tabelle 6 angegeben.

Einfluss der Temperatur auf den Reaktionsfortschritt

Um eine möglichst hohe Reaktionsgeschwindigkeit, aber dennoch selektive Reaktion zu erreichen wurden Versuche bei 40-70°C durchgeführt (Fig. 8). Mit Temperaturen bis 60°C steigt der Umsatz und die Ausbeute an NMM massiv an. Allerdings verringert sich die Ausbeute bei 70°C, was auf ein Durchgehen der Reaktion und dadurch eine Zersetzung der Edukte und auch des Produkts zwischen 60 und 70°C hindeutet. Im Gegensatz zu diskontinuierlichen Betriebsweisen erlaubt das erfindungsgemäße kontinuierliche Verfahren bei größeren Produktionsmengen eine exakte Temperaturkontrolle, sodass die Temperiertemperaturen auch im Reaktionsfluid gut eingehalten werden können. Parameter und Ergebnisse sind in Tabelle 7 angegeben.

Einfluss der Konzentration des Wasserstoffperoxids auf die Produktionsrate

Um zu ermitteln welche Konzentration an Wasserstoffperoxid in der Reaktionslösung sinnvoll ist wurden identische Versuche mit 30 und mit 50 Gew.%igem Wasserstoffperoxid durchgeführt (Fig. 9). Die Verwendung von 50 Gew.%igem Wasserstoffperoxid erwies sich als günstiger. Zudem kann so konzentrierteres NMMNO gewonnen werden. Der Vorteil des erfindungsgemäßen Mikroreaktionssystems ist, dass der Einsatz von sehr konzentriertem Wasserstoffperoxid kein Problem hinsichtlich der Wärmetönung ist. Parameter und Ergebnisse sind in Tabelle 8 angegeben.

Tabelle 7: Temperaturvariationen

| A | B | C | D | E | F | G | H | I | J | K | L | M | N | O | P | Q | R |
|---|---|---|---|---|---|---|---|---|---|---|---|---|---|---|---|---|---|
| Werks-toll | Temper-atur [°C] | Edukt 1 N-Me-thyl-Mor-pholin (NMM) [ml/min] | Edukt 2 Wasser-stoffper-oxid (H2O2) [ml/min] | Molver-hältnis Edukt 1 : Edukt 2 [mol/mol] | CO2 in NMM [Masse %] | Konzentration H2O2 [Masse %] | CO2 in H2O2 [Masse %] | H3PO4 In H2O2 [Masse %] | Druck [bar] | Durch-satz [ml/min] | Um-satz [%] | Aus-beute NMMO [%] | Fluidge-schwindigkeit [m/min] | hydrodyna-mische Ver-weilzeit Im Reaktions-bereich - Reaktor-länge [min] | Reaktor-spezi-tische Ober-flächen belas-tung [l/m²h] | Reaktor-spezi-tische Volu-mens belas-tung [l/m²h] | Ober-fläche: Volu-men Ver-hältnis |
| 1.4404 | 40 | 4,8 | 2,71 | 1:1,1 | 1 | 30 | 0 | 0,1 | 20 | 7,51 | 21 | 18 | 9,56 | 0,39 | 38,25 | 152.996,98 | 4 |
|  | 40 | 3,2 | 1,81 | 1:1,1 | 1 | 30 | 0 | 0,1 | 20 | 5,01 | 27 | 26 | 6,38 | 0,59 | 25,52 | 102.065,89 | 4 |
|  | 40 | 2,4 | 1,36 | 1:1,1 | 1 | 30 | 0 | 0,1 | 20 | 3,76 | 32 | 29 | 4,79 | 0,78 | 19,15 | 76.600,35 | 4 |
|  | 40 | 1,2 | 0,68 | 1:1,1 | 1 | 30 | 0 | 0,1 | 20 | 1,88 | 38 | 37 | 2,39 | 1,57 | 9,58 | 38.300,18 | 4 |
|  | 40 | 0,6 | 0,34 | 1:1,1 | 1 | 30 | 0 | 0,1 | 20 | 0,94 | 52 | 50 | 1,20 | 3,13 | 4,79 | 19.150,09 | 4 |
|  | 40 | 0,47 | 0,27 | 1:1,1 | 1 | 30 | 0 | 0,1 | 20 | 0,74 | 57 | 55 | 0,94 | 3,98 | 3,77 | 15.075,60 | 4 |
|  | 40 | 0,24 | 0,14 | 1:1,1 | 1 | 30 | 0 | 0,1 | 20 | 0,38 | 82 | 78 | 0,48 | 7,75 | 1,94 | 7.741,52 | 4 |
|  | 50 | 4,8 | 2,71 | 1:1,1 | 1 | 30 | 0 | 0,1 | 20 | 7,51 | 30 | 30 | 9,56 | 0,39 | 38,25 | 152.996,98 | 4 |
|  | 50 | 3,2 | 1,81 | 1:1,1 | 1 | 30 | 0 | 0,1 | 20 | 5,01 | 38 | 38 | 6,38 | 0,59 | 25,52 | 102.065,89 | 4 |
|  | 50 | 2,4 | 1,36 | 1:1,1 | 1 | 30 | 0 | 0,1 | 20 | 3,76 | 43 | 42 | 4,79 | 0,78 | 19,15 | 76.600,35 | 4 |
|  | 50 | 1,2 | 0,68 | 1:1,1 | 1 | 30 | 0 | 0,1 | 20 | 1,88 | 52 | 50 | 2,39 | 1,57 | 9,58 | 38.300,18 | 4 |
|  | 50 | 0,6 | 0,34 | 1:1,1 | 1 | 30 | 0 | 0,1 | 20 | 0,94 | 69 | 68 | 1,20 | 3,13 | 4,79 | 19.150,09 | 4 |
|  | 50 | 0,47 | 0,27 | 1:1,1 | 1 | 30 | 0 | 0,1 | 20 | 0,74 | 73 | 70 | 0,94 | 3,98 | 3,77 | 15.075,60 | 4 |
|  | 50 | 0,24 | 0,14 | 1:1,1 | 1 | 30 | 0 | 0,1 | 20 | 0,38 | 90 | 90 | 0,48 | 7,75 | 1,94 | 7.741,52 | 4 |

EP 3 339 288 A1

EP 3 339 288 A1

| A | B | C | D | E | F | G | H | I | J | K | L | M | N | O | P | Q | R |
|---|---|---|---|---|---|---|---|---|---|---|---|---|---|---|---|---|---|
| Werks-toll | Temper-atur [°C] | Edukt 1 N-Me-thyl-Mor-pholin (NMM) [ml/min] | Edukt 2 Wasser-stoff per-oxid (H2O2) [ml/min] | Molver-hältnis Edukt 1 : Edukt 2 [mol/mol] | CO2 in NMM [Masse %] | Konzentration H2O2 [Masse %] | CO2 in H2O2 [Masse %] | H3PO4 In H2O2 [Masse %] | Druck [bar] | Durch-satz [ml/min] | Um-satz [%] | Aus-beute NMMO [%] | Fluidge-schwindigkeit [m/min] | hydrodyna-mische Ver-weilzeit Im Reaktions-bereich - Reaktor-länge [min] | Reaktor-spezi-tische Ober-flächen belas-tung [l/m²h] | Reaktor-spezi-tische Volu-mens belas-tung [l/m²h] | Ober-fläche: Volumen Ver-hältnis |
| | 60 | 4,1 | 3,5 | 1:1,1 | 1 | 30 | 0 | 0,1 | 20 | 7,6 | 82 | 80 | 9,68 | 0,39 | 38,71 | 154.830,49 | 4 |
| | 60 | 2,7 | 2,3 | 1:1,1 | 1 | 30 | 0 | 0,1 | 20 | 5 | 83 | 83 | 6,37 | 0,59 | 25,47 | 101.862,17 | 4 |
| | 60 | 2 | 1,71 | 1:1,1 | 1 | 30 | 0 | 0,1 | 20 | 3,71 | 86 | 85 | 4,72 | 0,79 | 18,90 | 75.581,73 | 4 |
| | 60 | 1 | 0,85 | 1:1,1 | 1 | 30 | 0 | 0,1 | 20 | 1,85 | 84 | 85 | 2,36 | 1,59 | 9,42 | 37.689,00 | 4 |
| | 60 | 0,5 | 0,43 | 1:1,1 | 1 | 30 | 0 | 0,1 | 20 | 0,93 | 90 | 90 | 1,18 | 3,17 | 4,74 | 18.946,36 | 4 |
| | 60 | 0,4 | 0,34 | 1:1,1 | 1 | 30 | 0 | 0,1 | 20 | 0,74 | 88 | 87 | 0,94 | 3,98 | 3,77 | 15.075,60 | 4 |
| | 60 | 0,21 | 0,18 | 1:1,1 | 1 | 30 | 0 | 0,1 | 20 | 0,39 | 96 | 85 | 0,50 | 7,55 | 1,99 | 7.945,25 | 4 |
| | 70 | 4,8 | 2,71 | 1:1,1 | 1 | 30 | 0 | 0,1 | 20 | 7,51 | 35 | 30 | 9,56 | 0,39 | | 152.996,98 | 4 |
| | 70 | 3,2 | 1,81 | 1:1,1 | 1 | 30 | 0 | 0,1 | 20 | 5,01 | 25 | 22 | 6,38 | 0,59 | 25,52 | 102.065,89 | 4 |
| | 70 | 2,4 | 1,36 | 1:1,1 | 1 | 30 | 0 | 0,1 | 20 | 3,76 | 28 | 15 | 4,79 | 0,78 | 19,15 | 76.600,35 | 4 |
| | 70 | 1,2 | 0,68 | 1:1,1 | 1 | 30 | 0 | 0,1 | 20 | 1,88 | 25 | 23 | 2,39 | 1,57 | 9,58 | 38.300,18 | 4 |
| | 70 | 0,6 | 0,34 | 1:1,1 | 1 | 30 | 0 | 0,1 | 20 | 0,94 | 33 | 24 | 1,20 | 3,13 | 4,79 | 19.150,09 | 4 |
| | 70 | 0,47 | 0,27 | 1:1,1 | 1 | 30 | 0 | 0,1 | 20 | 0,74 | 28 | 22 | 0,94 | 3,98 | 3,77 | 15.075,60 | 4 |
| | 70 | 0,24 | 0,14 | 1:1,1 | 1 | 30 | 0 | 0,1 | 20 | 0,38 | 25 | 16 | 0,48 | 7,75 | 1,94 | 7.741,52 | 4 |

Tabelle 8: Parameter zur $H_2O_2$-Beigabe

| A | B | C | D | E | F | G | H | I | J | K | L | M | N | O | P | Q | R |
|---|---|---|---|---|---|---|---|---|---|---|---|---|---|---|---|---|---|
| | | Edukt 1 N-Me-thyl-Mor-pholin (NMM) [ml/min] | Edukt 2 Wasser-stoff per-oxid (H2O2) [ml/min] | Molver-hältnis Edukt 1 : Edukt 2 [mol/mol] | CO2 in NMM [Mas se %] | Konzen-tration H2O2 [Masse %] | CO2 In H2O2 [Mas se %] | H3PO 4 In H2O2 [Mas se %] | | Durch-satz [ml/min] | | | | hydrodyna-mische Ver-wellzelt Im Reaktions-bereich - Reaktor-länge [min] | Reaktor-spezi-tische Ober-flächen belas-tung [l/m²h] | Reaktor - spezi-fische Volu-mens belas-tung [l/m²h] | |
| Werks-toll | Temper-atur [°C] | | | | | | | | Druc k [bar] | | Um-satz [%] | Aus-beute NMMO [%] | Fluidge-schwindigkelt [m/min] | | | | Ober-fläche: Volu-men Ver-hältnis |
| 1.4404 | 60 | 4,8 | 2,71 | 1:1,1 | 1 | 30 | 0 | 0,1 | 20 | 7,51 | 39 | 38 | 9,56 | 0,39 | 38,2 | 152.997,0 | 4 |
| | 60 | 3,2 | 1,81 | 1:1,1 | 1 | 30 | 0 | 0,1 | 20 | 5,01 | 47 | 45 | 6,38 | 0,59 | 25,5 | 102.065,9 | 4 |
| | 60 | 2,4 | 1,36 | 1:1,1 | 1 | 30 | 0 | 0,1 | 20 | 3,76 | 51 | 45 | 4,79 | 0,78 | 19,2 | 76.600,4 | 4 |
| | 60 | 1,2 | 0,68 | 1:1,1 | 1 | 30 | 0 | 0,1 | 20 | 1,88 | 63 | 60 | 2,39 | 1,57 | 9,6 | 38.300,2 | 4 |
| | 60 | 0,6 | 0,34 | 1:1,1 | 1 | 30 | 0 | 0,1 | 20 | 0,94 | 79 | 75 | 1,20 | 3,13 | 4,8 | 19.150,1 | 4 |
| | 60 | 0,47 | 0,27 | 1:1,1 | 1 | 30 | 0 | 0,1 | 20 | 0,74 | 82 | 80 | 0,94 | 3,98 | 3,8 | 15.075,6 | 4 |
| | 60 | 0,24 | 0,14 | 1:1,1 | 1 | 30 | 0 | 0,1 | 20 | 0,38 | 93 | 90 | 0,48 | 7,75 | 1,9 | 7.741,5 | 4 |
| | 60 | 0,17 | 0,1 | 1:1,1 | 1 | 30 | 0 | 0,1 | 20 | 0,27 | 98 | 95 | 0,34 | 10,91 | 1,4 | 5.500,6 | 4 |
| | 60 | 4,8 | 2,71 | 1:1,1 | 1 | 50 | 0 | 0,1 | 20 | 7,51 | 47 | 45 | 9,56 | 0,39 | 38,2 | 152.997,0 | 4 |
| | 60 | 3,2 | 1,81 | 1:1,1 | 1 | 50 | 0 | 0,1 | 20 | 5,01 | 57 | 55 | 6,38 | 0,59 | 25,5 | 102.065,9 | 4 |
| | 60 | 2,4 | 1,36 | 1:1,1 | 1 | 50 | 0 | 0,1 | 20 | 3,76 | 63 | 60 | 4,79 | 0,78 | 19,2 | 76.600,4 | 4 |
| | 60 | 1,2 | 0,68 | 1:1,1 | 1 | 50 | 0 | 0,1 | 20 | 1,88 | 72 | 70 | 2,39 | 1,57 | 9,6 | 38.300,2 | 4 |
| | 60 | 0,6 | 0,34 | 1:1,1 | 1 | 50 | 0 | 0,1 | 20 | 0,94 | 86 | 84 | 1,20 | 3,13 | 4,8 | 19.150,1 | 4 |
| | 60 | 0,47 | 0,27 | 1:1,1 | 1 | 50 | 0 | 0,1 | 20 | 0,74 | 87 | 84 | 0,94 | 3,98 | 3,8 | 15.075,6 | 4 |
| | 60 | 0,24 | 0,14 | 1:1,1 | 1 | 50 | 0 | 0,1 | 20 | 0,38 | 97 | 97 | 0,48 | 7,75 | 1,9 | 7.741,5 | 4 |
| | 60 | 0,17 | 0,1 | 1:1,1 | 1 | 50 | 0 | 0,1 | 20 | 0,27 | 97 | 95 | 0,34 | 10,91 | 1,4 | 5.500,6 | 4 |
| | 60 | 4,8 | 2,71 | 1:1.1 | 0,5 | 50 | 0 | 0,1 | 20 | 7,51 | 38 | 34 | 9,56 | 0,39 | 38,2 | 152.997,0 | 4 |
| | 60 | 3,2 | 1,81 | 1:1,1 | 0,5 | 50 | 0 | 0,1 | 20 | 5,01 | 34 | 33 | 6,38 | 0,59 | 25,5 | 102.065,9 | 4 |
| | 60 | 2,4 | 1,36 | 1:1,1 | 0,5 | 50 | 0 | 0,1 | 20 | 3,76 | 54 | 53 | 4,79 | 0,78 | 19,2 | 76.600,4 | 4 |
| | 60 | 1,2 | 0,68 | 1:1,1 | 0,5 | 50 | 0 | 0,1 | 20 | 1,88 | 60 | 42 | 2,39 | 1,57 | 9,6 | 38.300,2 | 4 |

(fortgesetzt)

| A | B | C | D | E | F | G | H | I | J | K | L | M | N | O | P | Q | R |
|---|---|---|---|---|---|---|---|---|---|---|---|---|---|---|---|---|---|
| Werks-toll | Temper-atur [°C] | Edukt 1 N-Me-thyl-Mor-pholin (NMM) [ml/min] | Edukt 2 Wasser-stoff per-oxid (H2O2) [ml/min] | Molver-hältnis Edukt 1 : Edukt 2 [mol/mol] | CO2 in NMM [Mas se %] | Konzen-tration H2O2 [Masse %] | CO2 In H2O2 [Mas se %] | H3PO 4 In H2O2 [Mas se %] | Druc k [bar] | Durch-satz [ml/min ] | Um-satz [%] | Aus-beute NMMO [%] | Fluidge-schwindigkelt [m/min] | hydrodyna-mische Ver-wellzelt Im Reaktions-bereich - Reaktor-länge [min] | Reaktor-spezi-tische Ober-flächen belas-tung [l/m²h] | Reaktor-spezi-fische Volu-mens belas-tung [l/m²h] | Ober-fläche: Volu-men Ver-hältnis |
| | 60 | 0,6 | 0,34 | 1:1,1 | 0,5 | 50 | 0 | 0,1 | 20 | 0,94 | 70 | 69 | 1,20 | 3,13 | 4,8 | 19.150,1 | 4 |
| | 60 | 0,47 | 0,27 | 1:1,1 | 0,5 | 50 | 0 | 0,1 | 20 | 0,74 | 81 | 80 | 0,94 | 3,98 | 3,8 | 15.075,6 | 4 |
| | 60 | 0,24 | 0,14 | 1:1,1 | 0,5 | 50 | 0 | 0,1 | 20 | 0,38 | 90 | 90 | 0,48 | 7,75 | 1,9 | 7.741,5 | 4 |
| | 60 | 0,17 | 0,1 | 1:1,1 | 0,5 | 50 | 0 | 0,1 | 20 | 0,27 | 99 | 95 | 0,34 | 10,91 | 1,4 | 5.500,6 | 4 |

Großtechnischer Reaktor

Es wurden weitere Experimente mit den gleichen Chemikalien und analoger Versuchsdurchführung wie in den bisherigen Beispielen nur mit größerem Reaktor durchgeführt.

Das Design des größeren Reaktors ist ähnlich dem oben beschriebenen Reaktordesign mit folgenden Änderungen. Die Länge der verwendeten Rohre betrug 2,8 m mit einem Innendurchmesser von 1,5 bis 4 mm, mit und ohne Quetschungen. Die Anzahl der verwendeten Rohre betrug 40. Die erzielte Produktionskapazität in diesem Experiment war von 1 kg/h bis 3 kg/h NMMNO bezogen auf 100%iges NMMNO. Durch diese Produktionskapazität war es nötig, anstatt des Heizthermostats (siehe Reaktorskizze (2) - (Lauda: ECO SILVER RE415)) einen Kühler zu installieren.

Es wurde bei leichtem Überdruck (0,3 barü) 1 Gew-% $CO_2$ in NMM gelöst und anschließend das NMM/$CO_2$ in den Vorlagebehälter (3) überführt. Nun wurde 0,1 Gew.-% $H_3PO_4$ in die $H_2O_2$ Lösung gegeben und in den Vorlagebehälter (4) geleert. Die weitere Vorgehensweise ist ident zu den vorhin beschriebenen Beispielen.

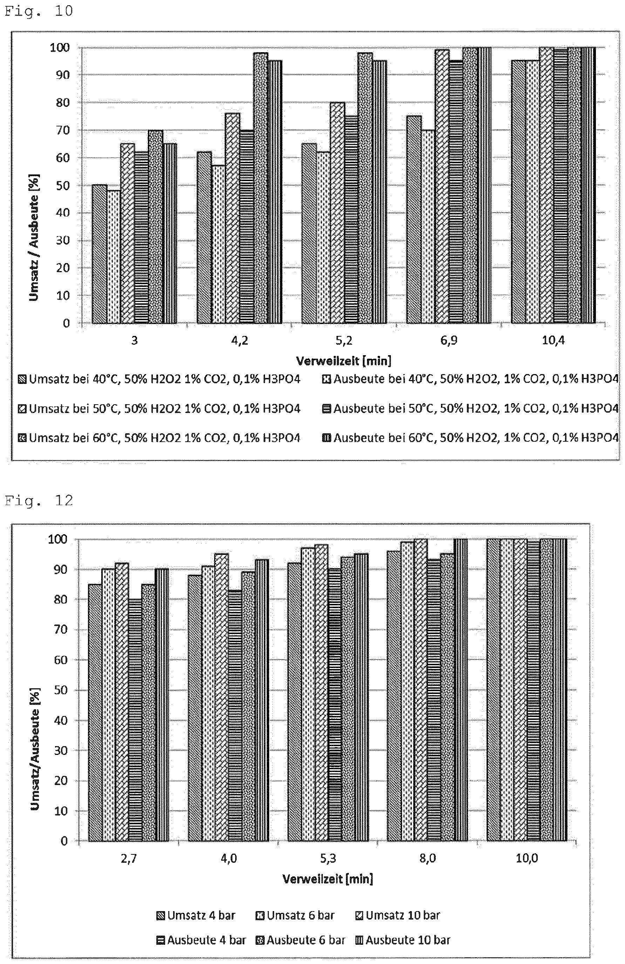

In diesem Experiment wurde versucht eine möglichst hohe Produktionskapazität durch den größeren Reaktor und durch größere Volumenströme zu erzielen. Ergebnisse sind in Fig. 10 und in Tabelle 9 gezeigt.

Tabelle 9: Großtechnischer Reaktor

| A Werkstoff | B Tempera-tur [°C] | C Edukt 1 N-Methyl-Morpholin (NMM) [ml/min] | D Edukt 2 Wasser-stoff peroxid (H2O2) [ml/min] | E Molverhältnis Edukt 1 : Edukt 2 [mol/mol] | F CO2 in NMM [Masse %] | G Konzentrati-on H2O2 [Masse %] | H CO2 in H2O2 [Masse %] | I H3PO4 in H2O2 [Masse %] | J Druck [bar] | K Durch-satz [ml/min] | L Um-satz [%] | M Ausbeu-te NMMO [%] | N max. Fluidge-schwindigkeit [m/min] | O hydrodynami-sche Verweilzeit im Reaktionsbe-reich [min] | P Reaktor-spezifische Oberflä-chen belastung [l/m²h] | Q Reaktor-spezifische Volumens belastung [l/m²h] | R Oberflä-che:Volumen Verhältnis |
|---|---|---|---|---|---|---|---|---|---|---|---|---|---|---|---|---|---|
| 1.4404 | 40 | 117,3 | 66,0 | 1:1,0 | 1 | 50,75 | 0 | 0,1 | 103 | 183,3 | 50 | 48 | 103,7 | 0,8 | 56,87 | 74445 | |
| | 40 | 83,8 | 47,1 | 1:1,1 | 1 | 50,75 | 0 | 0,1 | 68 | 130,9 | 62 | 57 | 74,1 | 1,1 | 40,61 | 53163 | |
| | 40 | 67 | 37,7 | 1:1,1 | 1 | 50,75 | 0 | 0,1 | 55 | 104,7 | 65 | 62 | 59,2 | 1,4 | 32,48 | 42523 | |
| | 40 | 50,3 | 28,3 | 1:1,1 | 1 | 50,75 | 0 | 0,1 | 43 | 78,6 | 75 | 70 | 44,5 | 1,9 | 24,39 | 31922 | |
| | 40 | 33,5 | 18,8 | 1:1,1 | 1 | 50,75 | 0 | 0,1 | 34 | 52,3 | 95 | 95 | 29,6 | 2,8 | 16,23 | 21241 | |
| | 50 | 117,3 | 66,0 | 1:1,0 | 1 | 50,75 | 0 | 0,1 | 95 | 183,3 | 65 | 62 | 103,7 | 0,8 | 56,87 | 74445 | |
| | 50 | 83,8 | 47,1 | 1:1,1 | 1 | 50,75 | 0 | 0,1 | 65 | 130,9 | 76 | 70 | 74,1 | 1,1 | 40,61 | 53163 | |
| | 50 | 67 | 37,7 | 1:1,1 | 1 | 50,75 | 0 | 0,1 | 52 | 104,7 | 80 | 75 | 59,2 | 1,4 | 32,48 | 42523 | |
| | 50 | 50,3 | 28,3 | 1:1,1 | 1 | 50,75 | 0 | 0,1 | 46 | 78,6 | 99 | 95 | 44,5 | 1,9 | 24,39 | 31922 | |
| | 50 | 33,5 | 18,8 | 1:1,1 | 1 | 50,75 | 0 | 0,1 | 32 | 52,3 | 100 | 99 | 29,6 | 2,8 | 16,23 | 21241 | |
| | 60 | 117,3 | 66,0 | 1:1,0 | 1 | 50,75 | 0 | 0,1 | 92 | 183,3 | 70 | 65 | 103,7 | 0,8 | 56,87 | 74445 | |
| | 60 | 83,8 | 47,1 | 1:1,1 | 1 | 50,75 | 0 | 0,1 | 65 | 130,9 | 98 | 95 | 74,1 | 1,1 | 40,61 | 53163 | |
| | 60 | 67 | 37,7 | 1:1,1 | 1 | 50,75 | 0 | 0,1 | 50 | 104,7 | 98 | 95 | 59,2 | 1,4 | 32,48 | 42523 | |
| | 60 | 50,3 | 28,3 | 1:1,1 | 1 | 50,75 | 0 | 0,1 | 41 | 78,6 | 100 | 100 | 44,5 | 1,9 | 24,39 | 31922 | |
| | 60 | 33,5 | 18,8 | 1:1,1 | 1 | 50,75 | 0 | 0,1 | 28 | 52,3 | 100 | 100 | 29,6 | 2,8 | 16,23 | 21241 | |

einer Verringerung des Oberfläche:Volumen Verhältnisses von 2,7 auf 1 über die Reaktor-länge, wobei es bis zu Umsätzen von 50% sicher >2 ist.

**Reaktionssystem nach Fig. 11**

CO$_2$ Sättigung direkt im Mikroreaktor

Der Versuch wurde wie zuvor beschrieben mit dem kleinen Reaktor durchgeführt mit dem Unterschied, dass das Edukt 1 NMM unverdünnt 99%ig über eine computergesteuerte Pumpe (5) aus dem Vorratsbehälter (3) in den Mikroreaktor (1) gefördert wird und mit Kühlwasser auf ca. 20°C temperiert wird. Die maximal lösliche Gasmenge, welche aus den Löslichkeitsversuchen ermittelt wurde, wird über ein Dosierventil (14) aus der Gasflasche (15) in den Mikroreaktor geleitet. Der Druck im Abschnitt der CO$_2$ Sättigung wird auf [A] bar geregelt.

Es wurden zwei Heizthermostate (2, 16) ECO SILVER RE415 der Firma Lauda eingesetzt um den Mikroreaktor auf T1=20 °C bzw. T2=65 °C zu temperieren. Als Kühl- und Heizmedium wurde ein Gemisch aus Wasser/Ethylenglycol im Volumensverhältnis 1:1 eingesetzt.

Im Wasserstoffperoxid Vorratsbehälter (4) wird das Edukt 2, Wasserstoffperoxid, mit 0,1 Gew.-% H$_3$PO$_4$ versetzt, vorgelagert und mit einer Pumpe (6) in den Mikroreaktor (1) gefördert. Nach dem Sättigungsrohr (7) und Vorwärmrohr (8) werden die Edukte über ein Mischmodul T3 (9) zusammengeführt. Im Mischmodul wird ein statischer Mischer (z.B. Mischplättchen) eingesetzt, gemäß WO 2010/055034. Anschließend wird das Reaktionsgemisch in die Reaktionsrohre (10) geleitet, wo dann die Reaktion stattfindet. Zur Erhöhung und Regelung des Drucks im Reaktor wurde nach dem Reaktoraustritt ein Druckhalteventil (11) mit 20 bar eingesetzt. Der Einbau des Druckhalteventils sollte die Zerfalls- und Nebenreaktionen möglichst unterdrücken, da es im Zuge dieser unerwünschten Reaktionen zur Gasentwicklung kommt.

Das gewünschte Produkt wird nach einer Infrarotmessung (13) in einem Gefäß mit Rührwerk (12) aufgefangen. Überschüssiges Wasserstoffperoxid wird durch das Überleiten der Reaktionslösung über immobilisiertes MnO$_2$ zerstört.

Tabelle 10: Edukte der separaten CO$_2$-Zufuhr:

|  | Edukt 1 | Edukt 2 | Edukt 3 |
|---|---|---|---|
| Volumenstrom | [B] ml/min | [C] ml/min | [D] Gew.-% bezogen auf Edukt 1 |
| Molares Verhältnis | | 1:1,1 | |
| Zusammensetzung | 99 Vol.-%iges N-Metyhlmorpholin | 50 Gew.-%iges Wasserstoffperoxid mit 0,1 Gew.-% H$_3$PO$_4$ | CO$_2$ |

Durchsätze von [E] ml/min mit Umsätzen von [F]% und Ausbeuten von [G]% konnten erreicht werden. Ergebnisse sind in Fig. 12 und Tab. 12 gezeigt.

Tabelle 11: Parameter und Einheiten:

| | |
|---|---|
| Fluidgeschwindigkeit | [H] m/min |
| Hydrodynamische Verweilzeit im Reaktionsbereich | [I] min |
| Reaktorspezifische Oberflächenbelastung | [J] L/m$^2$h |
| Reaktorspezifische Volumensbelastung | [K] L/m$^3$h |
| Oberflächen:Volumen Verhältnis | [L] |

Tabelle 12: Ergebnisse der separaten $CO_2$-Zufuhr:

| A | B | C | D | E | F | G | H | I | J | K | L |
|---|---|---|---|---|---|---|---|---|---|---|---|
| Druck Im SättigungsAbschnitt [bar] | Edukt 1 N-Methyl-Morpholln (NMM) [ml/min] | Edukt 2 Wasserstoff peroxid (H2O2) [ml/min] | CO2 in NMM [Masse %] | Durchsatz [ml/min] | Umsatz [%] | Ausbeute NMMO [%] | max. Fluidge-schwindigkelt [m/min] | hydrodyna-mische Ver-weilzelt Im Reak-tionsbereich [mln] | Reaktor - spezitische Ober-flächen be-lastung [l/m²h] | Reaktor - spezitische Volumens belastung [l/m³h] | Oberfläche: Volumen Verhältnis |
| 4 | 5,03 | 2,83 | 5 | 7,86 | 85 | 80 | 10,0 | 2,7 | 5,56 | 22457 | 4 |
| 4 | 3,35 | 1,88 | 5 | 5,23 | 88 | 83 | 6,7 | 4,0 | 3,70 | 14943 | 4 |
| 4 | 2,51 | 1,41 | 5 | 3,92 | 92 | 90 | 5,0 | 5,3 | 2,77 | 11200 | 4 |
| 4 | 1,68 | 0,9 | 5 | 2,6 | 96 | 93 | 3,3 | 8,0 | 1,85 | 7486 | 4 |
| 4 | 1,34 | 0,75 | 5 | 2,09 | 100 | 99 | 2,7 | 10,0 | 1,48 | 5971 | 4 |
| 6 | 5,03 | 2,83 | 7,5 | 7,86 | 90 | 85 | 10,0 | 2,7 | 5,56 | 22457 | 4 |
| 6 | 3,35 | 1,88 | 7,5 | 5,23 | 91 | 89 | 6,7 | 4,0 | 3,70 | 14943 | 4 |
| 6 | 2,51 | 1,41 | 7,5 | 3,92 | 97 | 94 | 5,0 | 5,3 | 2,77 | 11200 | 4 |
| 6 | 1,68 | 0,9 | 7,5 | 2,62 | 99 | 95 | 3,3 | 8,0 | 1,85 | 7486 | 4 |
| 6 | 1,34 | 0,75 | 7,5 | 2,09 | 100 | 100 | 2,7 | 10,0 | 1,48 | 5971 | 4 |
| 10 | 5,03 | 2,83 | 14 | 7,86 | 92 | 90 | 10,0 | 2,7 | 5,56 | 22457 | 4 |
| 10 | 3,35 | 1,88 | 14 | 5,23 | 95 | 93 | 6,7 | 4,0 | 3,70 | 14943 | 4 |
| 10 | 2,51 | 1,41 | 14 | 3,92 | 98 | 95 | 5,0 | 5,3 | 2,77 | 11200 | 4 |
| 10 | 1,68 | 0,9 | 14 | 2,62 | 100 | 100 | 3,3 | 8,0 | 1,85 | 7486 | 4 |
| 10 | 1,34 | 0,75 | 14 | 2,09 | 100 | 100 | 2,7 | 10,0 | 1,48 | 5971 | 4 |

**Patentansprüche**

1. Verfahren zur Herstellung eines Aminoxids durch Oxidation eines tertiären Amins in einem Reaktor mit einem länglichen Hohlkörper unter kontinuierlicher Einführung von tertiärem Amin in einem Reaktionsfluid und Ausführung von Aminoxid, wobei

   i) im Hohlkörper ein Oberfläche zu Volumenverhältnis von 0,5 $m^2/m^3$ oder mehr zumindest auf einer Länge des Hohlkörpers, in der das Amin zu 50% (mol.-%) oxidiert wird, vorhanden ist;
   ii) im Hohlkörper eine spezifische Oberflächenbelastung von 1 $l/m^2h$ bis 40 $l/m^2h$ vorhanden ist; und/oder
   iii) im Hohlkörper eine spezifische Volumenbelastung von 1.000 $l/m^3h$ bis 30.000 $l/m^3h$ vorhanden ist.

2. Verfahren nach Anspruch 1, **dadurch gekennzeichnet, dass** das tertiäre Amin vorzugsweise in einem Molverhältnis von 1:0,9 bis 1:1,3, vorzugsweise 1:1 bis 1:1,1 zum Wasserstoffperoxid als Oxidationsmittel eingesetzt wird.

3. Verfahren nach Anspruch 1 oder 2, **dadurch gekennzeichnet, dass** das Aminoxid N-Methyl-Morpholin-N-oxid und das tertiäre Amin N-Methylmorpholin sind.

4. Verfahren nach einem der Ansprüche 1 bis 3, **dadurch gekennzeichnet, dass** eine Strömungsgeschwindigkeit des Reaktionsfluids im Hohlkörper auf eine Verweilzeit des tertiären Amins von 0,4 Minuten bis 14 Minuten, bevorzugt 0,6 Minuten bis 8 Minuten, eingestellt wird.

5. Verfahren nach einem der Ansprüche 1 bis 4, **dadurch gekennzeichnet, dass** die Strömungsgeschwindigkeit des Reaktionsfluids 0,1 m/min bis 60 m/min, bevorzugt 10 m/min bis 40 m/min, am meisten bevorzugt 20 m/min bis 25 m/min, ist.

6. Verfahren nach einem der Ansprüche 1 bis 5, **dadurch gekennzeichnet, dass** die Reaktionsfluidtemperatur im Hohlkörper 20°C bis 70°C, bevorzugt 40°C bis 65°C, am meisten bevorzugt von 50°C bis 65°C, ist.

7. Verfahren nach einem der Ansprüche 1 bis 6, **dadurch gekennzeichnet, dass** der Druck im Hohlkörper 1 bar bis 200 bar, bevorzugt 5 bar bis 100 bar, am meisten bevorzugt 50 bar bis 100 bar, ist.

8. Verfahren nach einem der Ansprüche 1 bis 7, **dadurch gekennzeichnet, dass** der Innendurchmesser eines Hohlkörpers, vorzugsweise eine Rohres, des Reaktors 0,25 mm bis 10 mm ist, vorzugsweise 0,5 mm bis 6 mm, insbesondere bevorzugt 0,8 mm bis 4 mm, ist.

9. Verfahren nach einem der Ansprüche 1 bis 8, **dadurch gekennzeichnet, dass** die Länge eines Hohlkörpers, vorzugsweise Rohres, des Reaktors 0,5 m bis 20 m, bevorzugt 1 m bis 10 m, insbesondere 2 m bis 5 m, ist.

10. Verfahren nach einem der Ansprüche 1 bis 9, **dadurch gekennzeichnet, dass** eine Innenwand eines Hohlkörpers, vorzugsweise Rohres, des Reaktors ein oder mehrere Metalle enthält, vorzugsweise ausgewählt aus Fe, Cr, Ni, Mo, Co, Mn, Nb, Au, Pt, Ti oder Mischungen hiervon, wobei insbesondere bevorzugt eine Innenwand eines Hohlkörpers, vorzugsweise Rohres, aus austenitischem Stahl ist.

11. Verfahren nach einem der Ansprüche 1 bis 10, **dadurch gekennzeichnet, dass** das tertiäre Amin in einer Konzentration von 40 % bis 100%, vorzugsweise von 50% bis 99,5%, insbesondere 60 bis 98%, speziell bevorzugt 70% bis 97%, in den Hohlkörper zugeführt wird (alle % in vol.-%).

12. Verfahren nach einem der Ansprüche 1 bis 11, **dadurch gekennzeichnet, dass** ein Oxidationsmittel, vorzugsweise Wasserstoffperoxid, in einer Konzentration von 5 % bis 80%, vorzugsweise von 10% bis 70%, insbesondere 20 bis 60%, speziell bevorzugt 30% bis 55%, in den Hohlkörper zugeführt wird (alle % in gew.-%).

13. Verfahren nach einem der Ansprüche 1 bis 12, **dadurch gekennzeichnet, dass** das Reaktionsfluid bei einer Bodensteinzahlen von größer 10 , bevorzugt von 20 bis 200, gemäß der Formel

$$Bo = u * L/D_{ax},$$

worin Bo die Bodensteinzahl ist, u die Strömungsgeschwindigkeit des Reaktionsfluids, L die Länge des Hohlkörpers, vorzugsweise Rohres, des Reaktors und $D_{ax}$ der axiale Dispersionsstrom des Reaktionsfluids ist, durch den Hohlkörper befördert wird.

14. Verfahren nach einem der Ansprüche 1 bis 13, **dadurch gekennzeichnet, dass** $CO_2$, vorzugsweise in einer Menge von 0,5 Gew.-% bis 20 Gew.-% bezogen auf das tertiäre Amin, in den Hohlkörper zugeführt wird.

15. Verfahren nach einem der Ansprüche 1 bis 14, **dadurch gekennzeichnet, dass** ein Metallkomplexbildner, vorzugsweise ein Phosphat, dem Reaktionsfluid zugefügt wird, vorzugsweise in einer Menge von 0,01% bis 3%, vorzugsweise 0,03% bis 2%, speziell bevorzugt 0,05% bis 1,5% (alle Gew.-%).

16. Verfahren nach einem der Ansprüche 1 bis 15, **dadurch gekennzeichnet, dass** die Oxidation des tertiären Amins mit einem Oxidationsmittel durchgeführt wird und überschüssiges Oxidationsmittel abgebaut oder entfernt wird.

17. Verfahren nach einem der Ansprüche 1 bis 16, **dadurch gekennzeichnet, dass** das Aminoxid auf eine Konzentration zum Auflösen von Zellulose gebracht wird und optional weiters Zellulose darin aufgelöst wird und optional weiters die aufgelöste Zellulose in Endlosformkörper geformt wird und die Zellulose der Formkörper gefällt wird.

Fig. 1

Fig. 3

Fig. 2

Fig. 4

Fig. 5

Fig. 6

Umsatz / Ausbeute [%] vs Verweilzeit [min]

☒ Umsatz 60°Cohne H3PO4 Zusatz    ☒ Ausbeute 60°C ohne H3PO4 Zusatz

☒ Umsatz 60°C mit  H3PO4 Zusatz    ☒ Ausbeute 60°C mit H3PO4 Zusatz

Fig. 7

Umsatz / Ausbeute [%] vs Verweilzeit [min]

☒Ums. 50°C +  50% H2O2 0,5 CO2 +H3PO4    ☒Ums. 50°C +  50% H2O2 1,0 CO2 +H3PO4

☒Ums. 50°C +  50% H2O2 5,0 CO2 +H3PO4    ☒Ums. 50°C +  50% H2O2 10,0 CO2 +H3PO4

☷Ausb. 50°C +  50% H2O2 0,5 CO2 +H3PO4   ▥Ausb. 50°C +  50% H2O2 1,0 CO2 +H3PO4

■Ausb. 50°C +  50% H2O2 5,0 CO2 +H3PO4   ☷Ausb. 50°C +  50% H2O2 10,0 CO2 +H3PO4

Fig. 8

Fig. 9

Fig. 10

Fig. 12

Fig. 11

EP 3 339 288 A1

32

EDUKT 1

EDUKT 2

EDUKT 3

immobilisiertes MnO2

Katalase

PRODUKT

Europäisches
Patentamt

European
Patent Office

Office européen
des brevets

## EUROPÄISCHER RECHERCHENBERICHT

**Nummer der Anmeldung**

EP 16 20 6623

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 12 21 645 B (DU PONT DE NEMOURS) 28. Juli 1966 (1966-07-28) | 1,2,4-15 | INV. C07C291/04 |
| Y | * Spalte 3, Zeile 55 - Spalte 4, Zeile 3; Anspruch 1 * | 3,16,17 | C07D295/24 |
| | ----- | | |
| Y,D | US 3 447 939 A (D.L. JOHNSON) 3. Juni 1969 (1969-06-03) | 3,16 | |
| A | * Ansprüche 10, 17; Beispiel 1 * | 1,2, 4-15,17 | |
| | ----- | | |
| Y,D | DE 36 18 352 A1 (HÜLS) 3. Dezember 1987 (1987-12-03) | 3 | |
| A | * Spalte 2, Zeilen 54-55; Anspruch 1; Beispiel * | 1,2,4-17 | |
| | ----- | | |
| A,D | WO 2010/055034 A1 (ONEA ENGINEERING AUSTRIA, ET AL.) 20. Mai 2010 (2010-05-20) * Seiten 29-30; Anspruch 1 * | 1 | |
| | ----- | | |
| Y,D | WO 2013/030400 A1 (AUROTEC) 7. März 2013 (2013-03-07) * Anspruch 1 * | 17 | |
| | ----- | | |

**RECHERCHIERTE SACHGEBIETE (IPC)**

C07C
C07D

Der vorliegende Recherchenbericht wurde für alle Patentansprüche erstellt

| Recherchenort | Abschlußdatum der Recherche | Prüfer |
|---|---|---|
| Den Haag | 13. Juni 2017 | English, Russell |

KATEGORIE DER GENANNTEN DOKUMENTE

X : von besonderer Bedeutung allein betrachtet
Y : von besonderer Bedeutung in Verbindung mit einer
 anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
 nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes
 Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 20 6623

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-06-2017

| Im Recherchenbericht angeführtes Patentdokument | | Datum der Veröffentlichung | Mitglied(er) der Patentfamilie | | | Datum der Veröffentlichung |
|---|---|---|---|---|---|---|
| DE 1221645 | B | 28-07-1966 | DE | 1221645 | B | 28-07-1966 |
| | | | GB | 989420 | A | 14-04-1965 |
| | | | US | 3215741 | A | 02-11-1965 |
| US 3447939 | A | 03-06-1969 | BE | 703203 | A | 15-01-1968 |
| | | | BE | 703240 | A | 15-01-1968 |
| | | | DE | 1694047 | A1 | 30-10-1969 |
| | | | DE | 1694048 | A1 | 05-02-1970 |
| | | | FR | 1556993 | A | 14-02-1969 |
| | | | GB | 1144048 | A | 05-03-1969 |
| | | | GB | 1144759 | A | 12-03-1969 |
| | | | US | 3447939 | A | 03-06-1969 |
| | | | US | 3508941 | A | 28-04-1970 |
| DE 3618352 | A1 | 03-12-1987 | CA | 1279644 | C | 29-01-1991 |
| | | | DE | 3618352 | A1 | 03-12-1987 |
| | | | EP | 0254803 | A2 | 03-02-1988 |
| | | | FI | 872367 | A | 01-12-1987 |
| | | | JP | S62292775 | A | 19-12-1987 |
| | | | NO | 872252 | A | 01-12-1987 |
| | | | SU | 1456011 | A3 | 30-01-1989 |
| | | | US | 4748241 | A | 31-05-1988 |
| WO 2010055034 | A1 | 20-05-2010 | EP | 2184103 | A1 | 12-05-2010 |
| | | | EP | 2349552 | A1 | 03-08-2011 |
| | | | JP | 2012508109 | A | 05-04-2012 |
| | | | JP | 2016041426 | A | 31-03-2016 |
| | | | US | 2011224463 | A1 | 15-09-2011 |
| | | | WO | 2010055034 | A1 | 20-05-2010 |
| WO 2013030400 | A1 | 07-03-2013 | BR | 112014004632 | A2 | 14-03-2017 |
| | | | CN | 104080959 | A | 01-10-2014 |
| | | | CN | 202509164 | U | 31-10-2012 |
| | | | EP | 2565304 | A1 | 06-03-2013 |
| | | | EP | 2751312 | A1 | 09-07-2014 |
| | | | KR | 20140058672 | A | 14-05-2014 |
| | | | RU | 2014112330 | A | 10-10-2015 |
| | | | US | 2014367896 | A1 | 18-12-2014 |
| | | | WO | 2013030400 | A1 | 07-03-2013 |
| | | | ZA | 201401446 | B | 29-04-2015 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013030400 A
- US 2179181 A
- US 3447939 A
- DE 2557456 A1
- DD 246997 A1
- DE 3618352 A1
- EP 0307184 A2

- US 4247480 A
- US 4994614 A
- US 5216154 A
- WO 2010055034 A
- EP 0448924 B1
- DE 3504899 A1